# EUROPÄISCHE PATENTSCHRIFT

(11) **EP 3 156 031 B1**
(45) Veröffentlichungstag und Bekanntmachung des Hinweises auf die Patenterteilung: **28.08.2019**
(21) Anmeldenummer: 15189615.6
(22) Anmeldetag: 13.10.2015
(51) Int. Cl.: A61K 6/00, A61K 6/083, A61L 24/06, C08L 33/08, C08L 33/10

(54) **POLYMERISIERBARE ZUSAMMENSETZUNGEN AUF BASIS VON THERMISCH SPALTBAREN VERBINDUNGEN**
POLYMERISABLE COMPOSITIONS BASED ON THERMALLY CLEAVABLE COMPOUNDS
COMPOSITIONS POLYMERISABLES A BASE DE LIAISONS FISSIBLES THERMIQUEMENT

(43) Veröffentlichungstag der Anmeldung: 19.04.2017
(73) Patentinhaber: Ivoclar Vivadent AG, 9494 Schaan (LI); Karlsruher Institut für Technologie, 76131 Karlsruhe (DE)
(72) Erfinder: Moszner, Norbert, 9495 Triesen (LI); Lamparth, Iris, 9472 Grabs (CH); Rist, Kai, 6800 Feldkirch (AT); Barner-Kowollik, Christoph, 4059 Kenmore Queensland (AU); Schenzel, Alexander, 76131 Karlsruhe (DE); Langer, Marcel, 69242 Mühlhausen (DE)
(74) Vertreter: Uexküll & Stolberg

(56) Entgegenhaltungen:
- US-A1- 2007 142 494
- US-A1- 2014 329 929
- US-A1- 2014 371 341
- Römpp Chemie Lexikon: 1 January 2015 (2015-01-01), Georg Thieme Verlag, Stuttgart
- SCHENZEL AM ET AL: "Reversing Adhesion: A Triggered Release Self-Reporting Adhesive", ADVANCED SCIENCE, vol. 3, 1500361, 2 February 2016 (2016-02-02), DOI: 10.1002/advs.201500361

## Beschreibung

Die vorliegende Erfindung betrifft thermisch spaltbare Verbindungen und deren Verwendung als vernetzende Monomerkomponenten insbesondere in Polymerisationsharzen und Polymerisaten wie Adhäsiven, Kompositen, Stereolithographiewerkstoffen, Formteilen, Duromeren sowie chemisch reversiblen Montage- und Befestigungselementen und insbesondere in Dentalwerkstoffen wie Adhäsiven, Zementen und Füllungskompositen.

Wiederlösbare Klebeverbindungen haben in verschiedenen Bereichen der Technik eine zunehmende Bedeutung. Beispiele sind das Entfügen von Bauteilen im Rahmen von automatisierten Fertigungsprozessen, die Reparatur von komplexen Bauteilen mit geklebten Teilkomponenten oder die Vereinfachung der stofflichen Trennung beim Recycling solcher Bauteile am Produktlebensende. Das Lösen (debonding) von Klebeverbindungen kann gezielt (on demand) dadurch erreicht werden, dass die Festigkeit der adhäsiven Verbundschicht z.B. durch Erwärmung deutlich verringert wird.

So beschreibt DE 198 32 629 A1 ein Klebstoffsystem zur Bildung reversibler Klebeverbindungen auf Basis von Polyurethanen, Polyharnstoffen oder Epoxidharzen, bei der eine Zusatzkomponente durch Energieeintrag so aktiviert werden kann, dass ein Abbau der Klebstoffkomponenten erfolgt. Beispielsweise können durch Eintrag von Wärme- oder Strahlungsenergie aus blockierten Precursoren organische Basen oder Säuren freigesetzt werden, die einen Abbau des Kleberharzes bewirken.

WO 2010/128042 A1 beschreibt technische Klebstoffzusammensetzungen für lösbare Klebeverbindungen für den Flugzeug- oder Fahrzeugbau, die aus einer üblichen Klebstoffmatrix und einem partikelförmigen Expansionsstoff wie z.B. Azodicarbonamid bestehen. Dabei erfolgt das Lösen der Bauteile durch Erwärmen der Klebeverbindung mindestens auf die Expansionstemperatur des Expansionsstoffes.

Im Dentalbereich ist das Lösen von Klebeverbindungen unter anderem in der Kieferorthopädie von Bedeutung, wo Brackets, die zur Korrektur von Zahnfehlstellungen auf die Zahnoberfläche geklebt werden, nach erfolgter Korrektur ohne Schädigung des Zahnschmelz wieder entfernen werden müssen. Ausserdem wären im Falle der Reparatur oder des vollkommenen Ersatzes von hochfesten keramischen Restaurationen oder Kronen, die mechanisch nur aufwändig entfernbar sind, einfach erweichbare bzw. trennbare Zementverbunde von Vorteil.

Im Zusammenhang mit orthodontischen Anwendungen beschreibt US 2007/ 0142498 A1 dentale Zusammensetzungen, die thermisch steuerbare Additive wie z.B. thermoplastische Polymere enthalten.

US 2007/0142497 A1 beschreibt dentale Zusammensetzungen auf Basis von Dimethacrylaten mit säurelabilen tertiären Carbonatgruppen und Photosäuren wie z.B. Triarylsulfoniumsalzen. Diese Zusammensetzungen lassen sich mit geeigneten Initiatoren wie etwa dem Bisacylphosphinoxid Irgacure 819 mit Licht im sichtbaren Bereich photochemisch härten (Photobonding) und durch Bestrahlung mit UV-Licht bei erhöhter Temperatur wieder erweichen (photothermisches Debonding) .

Die US 2007/0142494 A1 offenbart dentale Zusammensetzungen, die thermolabile Verbindungen mit einer oder mehreren thermolabilen Gruppen enthalten. Durch Erhitzen kann die Haftung der Zusammensetzungen am Zahn verringert werden. Die thermolabilen Verbindungen können einen Oximester oder ein Cycloadditionsaddukt enthalten, beispielsweise ein Diels-Alder-Addukt.

WO 2013/034777 A2 beschreibt Dentalmaterialien, die polymerisierbare Verbindungen mit einer thermolabilen oder photolabilen Gruppe wie etwa einer thermolabilen Diels-Alder-Gruppe enthalten. Es hat sich jedoch gezeigt, dass diese Materialien zur Bewirkung einer ausreichend schnellen Erweichung relativ hohen Temperaturen von typischerweise mehr als 100 °C ausgesetzt werden müssen.

Der Erfindung liegt die Aufgabe zugrunde, Zusammensetzungen bereitzustellen, die bei Raumtemperatur lagerstabil und unter Bildung von Netzwerkpolymeren polymerisierbar sind und im polymerisierten Zustand bereits bei relativ niedrigen Temperaturen von insbesondere weniger als 100 °C reversibel thermisch erweichbar sind und sich damit vor allem zur Herstellung von Materialien mit selbstheilenden oder Debonding-on-Demand-Eigenschaften wie chemisch reversiblen Montage- und Befestigungselementen sowie von Dentalwerkstoffen wie Adhäsiven, Zementen und Füllungskompositen eignen.

Diese Aufgabe wird erfindungsgemäss durch polymerisierbare Zusammensetzungen auf Basis einer thermolabilen polymerisierbaren Verbindung der Formel I gelöst:

A-[X-(T-X-Sp-X)ₚ-T-X-Q-(Z)ₙ]ₘ Formel I,

in der
- A: für einen m-wertigen linearen, verzweigten oder cyclischen aliphatischen oder aromatischen C₁-C₃₀-Rest, der durch -0-, -S-, -CO-O-, -O-CO-, -CO-NR³-, -NR³-CO-, -O-CO-NR³-, -NR³-CO-O-oder -NR³-CO-NR³- unterbrochen sein kann, oder eine oligo-mere Gruppe mit einer Molmasse von 200 bis 2000 g/mol steht,
- Q: jeweils unabhängig entfällt oder für einen (n+1)-wertigen linearen, verzweigten oder cyclischen aliphatischen oder aromatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR3- unterbrochen sein kann,
- Sp: jeweils unabhängig für einen linearen, verzweigten oder cyclischen aliphatischen oder aromatischen C₁-C₃₀-Rest, der durch -O-, -S-, -CO-O-, -O-CO-, -CO-NR³-, -NR³-CO-, -O-CO-NR³-, -NR³-CO-O- oder -NR³-CO-NR³ unterbrochen sein kann, oder eine oligomere Gruppe mit einer Molmasse von 200 bis 2000 g/mol steht,
- T: jeweils unabhängig ausgewählt ist aus 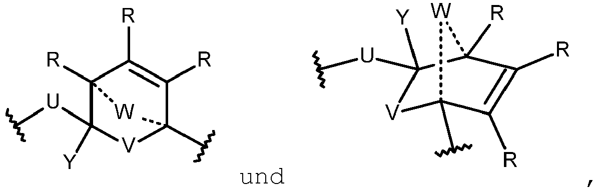
- R: jeweils unabhängig für H oder einen C₁-C₇-Alkyl-Rest steht,
- U: für S steht,
- V: für S steht,
- W: jeweils unabhängig entfällt oder für CH₂, 0, S oder NH steht,
- X: jeweils unabhängig entfällt oder für -O-, -S-, -CO-O-, -O-CO-, -CO-NR³-, -NR³-CO-, -O-CO-NR³-, -NR³-CO-O- oder -NR³-CO-NR³- steht,
- Y: jeweils unabhängig für H, R⁴, Aryl, Heteroaryl, -CN, -OR⁴, -N(R⁴)₂ oder -P(O)(OR⁴)₂ steht,
- Z: jeweils unabhängig für eine polymerisierbare Gruppe ausgewählt aus Vinylgruppen, CH₂=CR¹-CO-O- und CH₂=CR¹-CO-NR²-steht,
- R¹, R² und R³: jeweils unabhängig für H oder einen linearen oder verzweigten C₁-C₉-Alkyl-Rest stehen,
- R⁴: jeweils unabhängig für einen linearen oder verzweigten C₁-C₉-Alkyl-Rest steht,
- m: die Werte 2 bis 6 annehmen kann,
- n: jeweils unabhängig die Werte 1, 2 oder 3 annehmen kann,
- p: die Werte 0 bis 10 annehmen kann.

Der Hinweis, dass ein Rest durch eine Gruppe wie beispielsweise -0- unterbrochen sein kann, ist so zu verstehen, dass die Gruppe in die Kohlenstoffkette des Restes eingeschoben wird, d.h. beidseitig von Kohlenstoffatomen begrenzt wird. Die Anzahl dieser Gruppen ist daher um mindestens 1 kleiner als die Zahl der Kohlenstoffatome, und die Gruppen können nicht endständig sein. Erfindungsgemäss sind Reste, die nicht durch die genannten Gruppen unterbrochen sind, bevorzugt.

Dabei werden nur solche Verbindungen in Erwägung gezogen, die mit der chemischen Valenzlehre vereinbar sind.

Erfindungsgemäss sind Verbindungen der Formel I bevorzugt, bei denen unabhängig voneinander
- A: für einen m-wertigen linearen oder verzweigten aliphatischen oder aromatischen C₂-C₂₀-Rest, insbesondere C₄-C₁₈-Rest, bevorzugt C₈-C₁₆-Rest und besonders bevorzugt C₁₀-C₁₄-Rest, der durch -O-, -CO-O-, -O-CO-, -CO-NR³-, -NR³-CO-, -O-CO-NR³-oder -NR³-CO-O- unterbrochen sein kann, oder eine oligomere Gruppe mit einer Molmasse von 400 bis 1000 g/mol steht,
- Q: jeweils unabhängig entfällt oder für einen (n+1)-wertigen linearen oder verzweigten aliphatischen oder aromatischen C₂-C₁₅-Rest, insbesondere C₃-C₁₂-Rest und bevorzugt C₅-C₁₀-Rest steht, der durch -O-, -CO-O-, -O-CO-, -CO-NR³-, -NR³-CO-, -O-CO-NR³- oder -NR³-CO-O- unterbrochen sein kann, und besonders bevorzugt für -CH₂-Phenylen-CO-O-CH₂-CH₂- steht,
- Sp: jeweils unabhängig für einen linearen oder verzweigten aliphatischen oder aromatischen C₂-C₂₀-Rest, insbesondere C₄-C₁₈-Rest, bevorzugt C₈-C₁₆-Rest und besonders bevorzugt C₁₀-C₁₄-Rest, der durch -O-, -CO-O-, -O-CO-, -CO-NR³-, -NR³-CO-, -O-CO-NR³- oder -NR³-CO-O- unterbrochen sein kann, oder eine oligomere Gruppe mit einer Molmasse von 400 bis 1000 g/mol steht,
- X: jeweils unabhängig entfällt oder für -O-, -CO-O-, -O-CO-, -CO-NR³-, -NR³-CO-, -O-CO-NR³- oder -NR³-CO-O- steht,
- Z: jeweils unabhängig für eine polymerisierbare Gruppe ausgewählt aus CH₂=CR¹-CO-O- und CH₂=CR¹-CO-NR²- steht,
- R¹, R² und R³: jeweils unabhängig für H oder einen C₁-C₇-Alkyl-Rest, insbesondere H oder einen C₁-C₅-Alkyl-Rest und bevorzugt H oder einen C₁-C₃-Alkyl-Rest stehen,
- R⁴: jeweils unabhängig für einen C₁-C₇-Alkyl-Rest, insbesondere einen C₁-C₅-Alkyl-Rest, bevorzugt einen C₁-C₃-Alkyl-Rest und besonders bevorzugt für Methyl, Ethyl oder Isopropyl steht,
- m: die Werte 2, 3 oder 4 annehmen kann und bevorzugt 2 ist,
- n: jeweils unabhängig die Werte 1 oder 2 annehmen kann und bevorzugt 1 ist und
- p: die Werte 0, 1, 2 oder 3 annehmen kann, insbesondere 0 oder 1 ist und bevorzugt 0 ist.

Insbesondere sind solche Verbindungen der Formel I bevorzugt, bei denen
- R: jeweils unabhängig für H oder einen C₁-C₅-Alkyl-Rest, bevorzugt H oder einen C₁-C₃-Alkyl-Rest und besonders bevorzugt für H steht.

Ganz besonders sind solche Verbindungen der Formel I bevorzugt, bei denen jeweils unabhängig voneinander
- W: entfällt oder für CH₂ oder O steht und
- Y: jeweils unabhängig für Heteroaryl oder -P(O)(OR⁴)₂ steht und insbesondere für Pyridyl oder -P(O) (OEt)₂ steht.

Besonders bevorzugt sind jeweils solche Gruppen, in denen alle Variablen jeweils eine der oben definierten bevorzugten Bedeutungen haben.

Es wurde überraschend gefunden, dass die erfindungsgemässen Zusammensetzungen, die mindestens eine thermolabile polymerisierbare Verbindung der Formel I enthalten, sich sehr gut radikalisch zu Netzwerkpolymeren polymerisieren lassen, die bei Raumtemperatur lagerstabil sind, aber bereits bei mässig erhöhten Temperaturen von insbesondere weniger als 100 °C eine deutliche reversible thermische Spaltung zeigen. Diese Zusammensetzungen eignen sich daher in besonderer Weise zur Herstellung von Materialien mit selbstheilenden oder Debonding-on-Demand-Eigenschaften, vor allem zur Herstellung von Polymerisationsharzen und Polymerisaten wie Adhäsiven, Kompositen, Stereolithographiewerkstoffen, Formteilen, Duromeren und Montage- und Befestigungselementen sowie zur Herstellung von Dentalwerkstoffen wie Adhäsiven, Zementen und Füllungskompositen.

Insbesondere eignen sich die erfindungsgemässen Zusammensetzungen zur Herstellung von Polymerisaten, die eine Halbwertstemperatur von weniger als 120 °C, insbesondere weniger als 110 °C, bevorzugt weniger als 100 °C und besonders bevorzugt weniger als 90 °C aufweisen. Dabei bezeichnet der Begriff Halbwertstemperatur diejenige Temperatur, bei der die Hälfte der in einer Probe enthaltenen thermolabilen Gruppen T in gespaltener Form vorliegen. Die Bestimmung der Halbwertstemperatur kann insbesondere anhand der spektroskopische Verfolgung der Retro-Diels-Alder-Reaktion mittels eines Spektrometers mit temperaturregulierbarem Probenhalter bei einer Aufheizrate von 10 K/min erfolgen.

Die erfindungsgemässen thermolabilen polymerisierbaren Verbindungen der Formel I können einfach hergestellt werden. So kann die Synthese erfindungsgemässer Hetero-Diels-Alder-Addukt-Gruppen (HDA-Addukt-Gruppen) nach folgender allgemeiner Formel erfolgen:

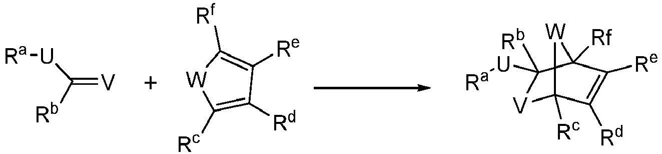

Konkretes Beispiel: Umsetzung eines Dithioesters mit Cyclopentadien:

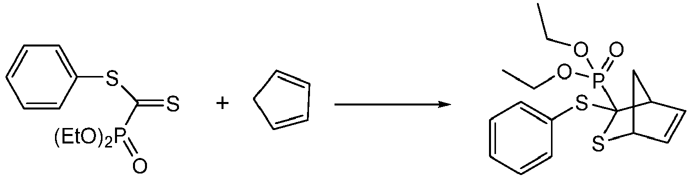

Werden Verbindungen mit Bis(dien)-Bausteinen mit Dienophilen, die polymerisationsfähige Gruppen enthalten, umgesetzt, so kommt man direkt zu Verbindungen der allgemeinen Formel I, wie z.B.:

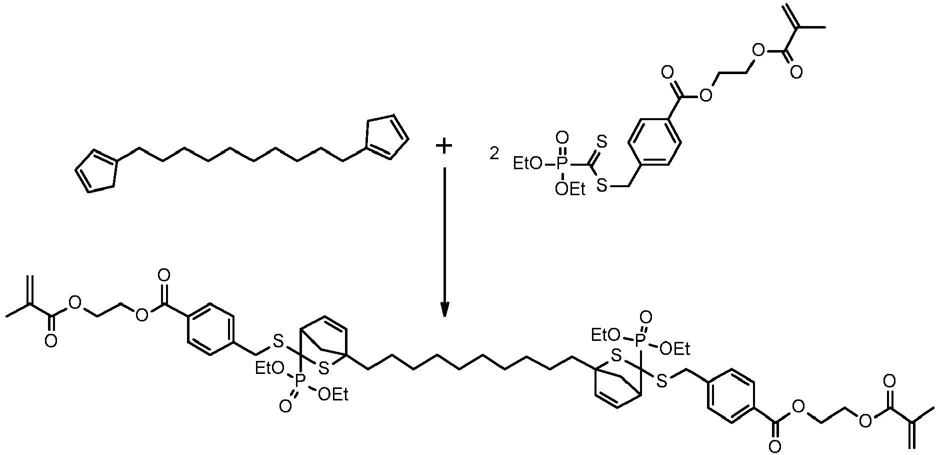

Bei der Reaktion von z.B. mit OH-Gruppen funktionalisierten Dien-Bausteinen (z.B. Furan-Derivat) und Dienophilen (z.B. Dithioester), kommt man direkt zu HDA-Addukten, die durch Reaktion mit geeigneten difunktionellen Verbindungen, z.B. im Falle der OH-funktionalisierten HDA-Addukte mit Diisocyanaten oder Dicarbonsäurederivaten, oligomerisiert werden können, wobei man für OH-terminierte Oligomere einen Überschuss an HDA-Addukt einsetzt:

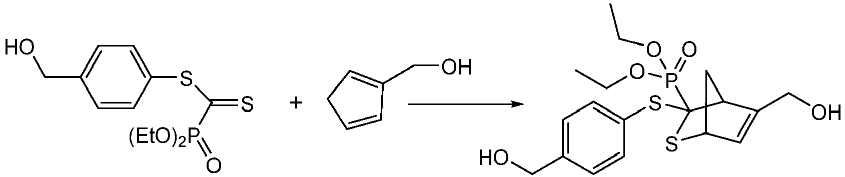

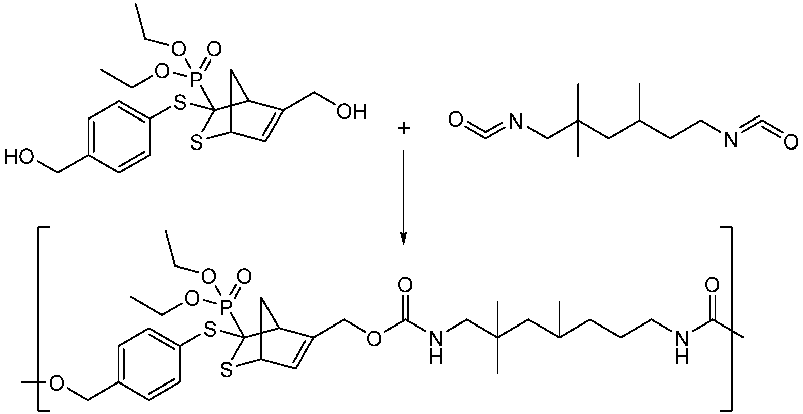

Durch anschliessende Endgruppenfunktionalisierung der multifunktionellen OH-terminierten HDA-Addukt-Oligomeren mit polymerisationsfähigen Gruppen sind dann die Verbindungen der Formel I zugänglich.

Geeignete Ausgangsstoffe für die Synthese der polymerisationsfähigen di- oder multifunktionellen Diels-Alder- oder Hetero-Diels-Alder-Addukte der allgemeinen Formel I sind geeignete Dien-Derivate, die sich vor allem von substituierten Butadien-1,3-, Cyclopentadien- (Cp) oder Furanderivaten ableiten. Eine besondere Klasse stellen die Photoenol-Derivate dar, da hier die Dien-Spezies erst in-situ gebildet wird. Photoenole lassen sich über eine mehrstufige Synthese aus Oxidation (D. M. Bauer, A. Rogge, L. Stolzer, C. Barner-Kowollik, L. Fruk, Chem. Commun. 2013, 49, 8626-8628), Etherspaltung (K. K. Oehlenschlaeger, J. O. Mueller, N. B. Heine, M. Glassner, N. K. Guimard, G. Delaittre, F. G. Schmidt, C. Barner-Kowollik, Angew. Chem. Int. Ed. 2013, 52, 762-766), Williamsonsche Ethersynthese (D. M. Bauer, A. Rogge, L. Stolzer, C. Barner-Kowollik, L. Fruk, Chem. Commun. 2013, 49, 8626-8628) und einer Verseifung (T. Pauloehrl, G. Delaittre, V. Winkler, A. Welle, M. Bruns, H. G. Börner, A. M. Greiner, M. Bastmeyer, C. Barner-Kowollik, Angew. Chem. Int. Ed. 2012, 51, 1071-1074) herstellen. Eine Reihe 1,3-Butadien-Derivate sind kommerziell erhältlich, wie das 2,4-Hexadien-1-ol oder die Sorbinsäure. Weitere Derivate lassen sich durch Additionsreaktionen (W. M. Gramlich, G. Theryo, M. A. Hillmyer, Polym. Chem. 2012, 3, 1510-1516) wie beim 2-Methylenebut-3-en-1-ol oder durch Substitutionsreaktionen (Y. Jing, V. V. Sheares, Macromolecules 2000, 33, 6255-6261) wie beim 3-Methylen-4-pentennitril synthetisieren. Geeignete Cp-Derivate lassen sich beispielsweise durch Substitutionsreaktionen aus den entsprechenden Brom-substituierten Derivaten mit NaCp (S. Bian, A. M. Scott, Y. Cao, Y. Liang, S. Osuna, K. N. Houk, A. B. Braunschweig, J. Am. Chem. Soc. 2013, 135, 9240-9243) oder NiCp₂ (M. Langer, J. Brandt, A. Lederer, A. S. Goldmann, F. H. Schacher, C. Barner-Kowollik, Polym. Chem. 2014, 5, 5330-5338) herstellen. Kommerziell erhältlich sind Cp-Derivate wie (1-Methyl-2,4-cyclopentadien-1-yl)methanol. Kommerziell zugängliche Furanderivate sind z.B. Furfural, Furfurylalkohol oder Brenzschleimsäure (vgl. Ullmann's Encyclopedia of Industrial Chemistry, 5th Ed. Vol. A 12, VCH, Weinheim etc. 1989, 119 ff.). Substituierte Furanderivate lassen sich durch Paal-Knorr-Synthese durch Erhitzen von entsprechenden 1,4-Diketoverbindungen herstellen (vgl. W. Walter, W. Francke, Beyer-Walter Lehrbuch der Organischen Chemie, S. Hirzel Verlag, Stuttgart und Leipzig 2004, 24. Aufl., S. 769).

Für die Synthese der polymerisationsfähigen di- oder multifunktionellen Hetero-Diels-Alder-Addukte der Formel I sind besonders geeignete Dienophile Thioester-Derivate, wie z.B. 4-((((Diethoxy-phosphoryl)carbonothioyl)thio)methyl)-benzoesäure oder 4-(((Pyridin-2-carbonothioyl)thio)methyl)-benzoesäure, die sich über Substitutionsreaktionen bzw. Salzmetathese herstellen lassen (A. Alberti, M. Benaglia, M. Laus, K. Sparnacci, J. Org. Chem. 2002, 67, 7911-7914). Thioaldehyde welche bei der photochemischen Spaltung von Phenacylsulfiden entstehen, können ebenfalls als Dienophile verwendet werden (M. Glassner, K. K. Oehlenschlaeger, A. Welle, M. Bruns, C. Barner-Kowollik, Chem. Commun. 2013, 49, 633-635).

Herstellen lassen sie sich durch die Reaktion von Phenacylbromiden mit Thiolen (G. A.-N. Gohar, S. N. Khattab, O. O. Farahat, H. H. Khalil, J. Phys. Org. Chem. 2012, 25, 343-350). Allgemein sind Dienophile mit einer elektronenziehenden Gruppe in Konjugation zur Doppelbindung wie z.B. substituierte Maleimide (A. S. Quick, H. Rothfuss, A. Welle, B. Richter, J. Fischer, M. Wegener, C. Barner-Kowollik, Adv. Funct. Mater. 2014, 24, 3571-3580) geeignet für (Hetero)-Diels-Alder Reaktionen.

Hetero-Diels-Alder Reaktionen laufen typischerweise unter Verwendung eines Katalysators wie z.B. ZnCl₂ (vgl. M. Langer, J. Brandt, A. Lederer, A. S. Goldmann, F. H. Schacher, C. Barner-Kowollik, Polym. Chem. 2014, 5, 5330-5338) oder TFA (A. J. Inglis, L. Nebhani, O. Altintas, F. G. Schmidt, C. Barner-Kowollik, Macromolecules 2010, 43, 5515-5520) oder auch katalysatorfrei ab (M. Glassner, G. Delaittre, M. Kaupp, J. P. Blinco, C. Barner-Kowollik, J. Am. Chem. Soc. 2012, 134, 7274-7277). Als Reaktionsmedium können sowohl organische Lösungsmittel als auch Wasser verwendet werden.

Beispiele für die erfindungsgemässen thermolabilen polymerisierbaren Verbindungen der Formel I sind:

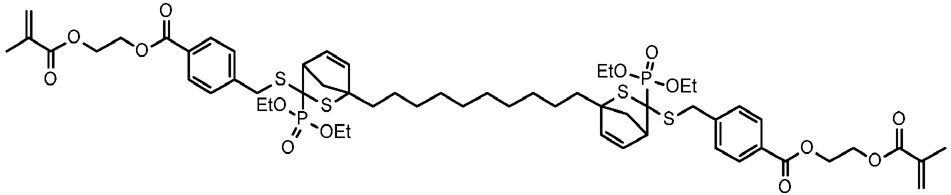

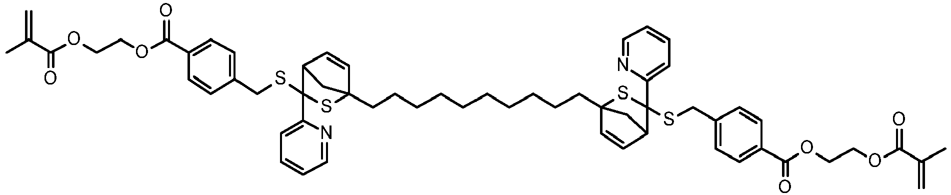

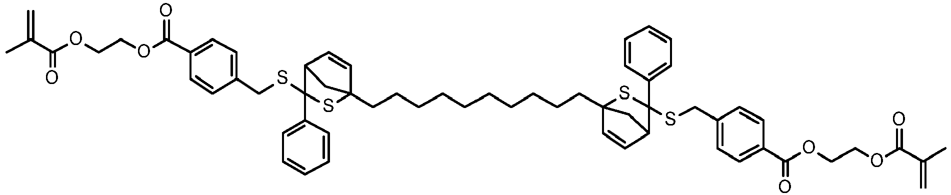

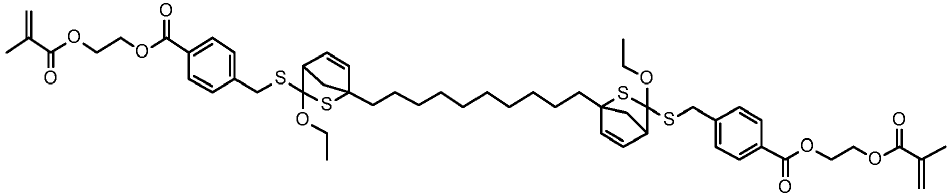

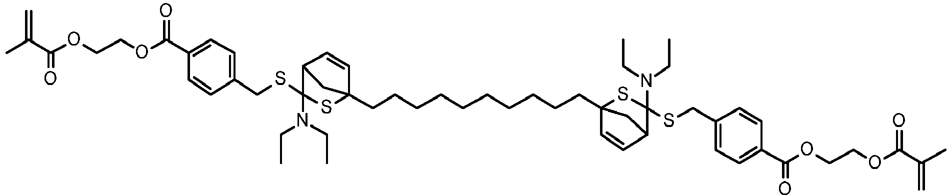

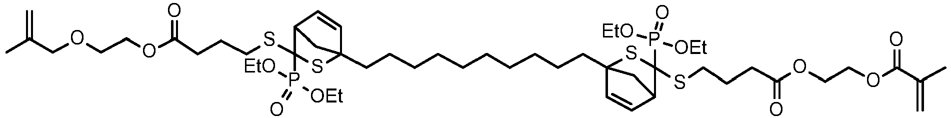

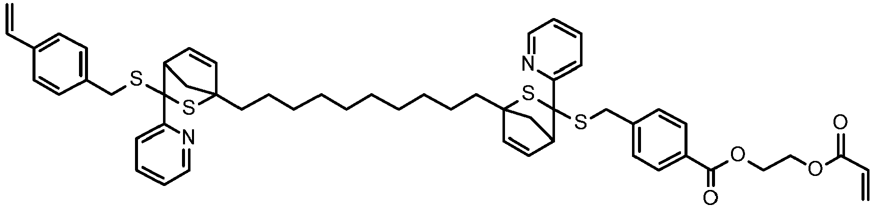

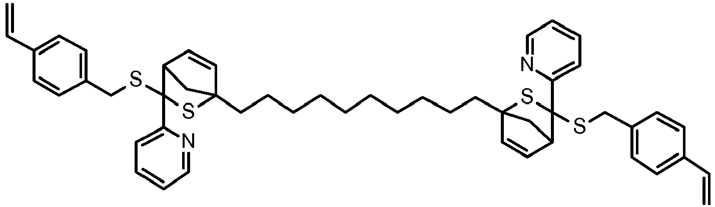

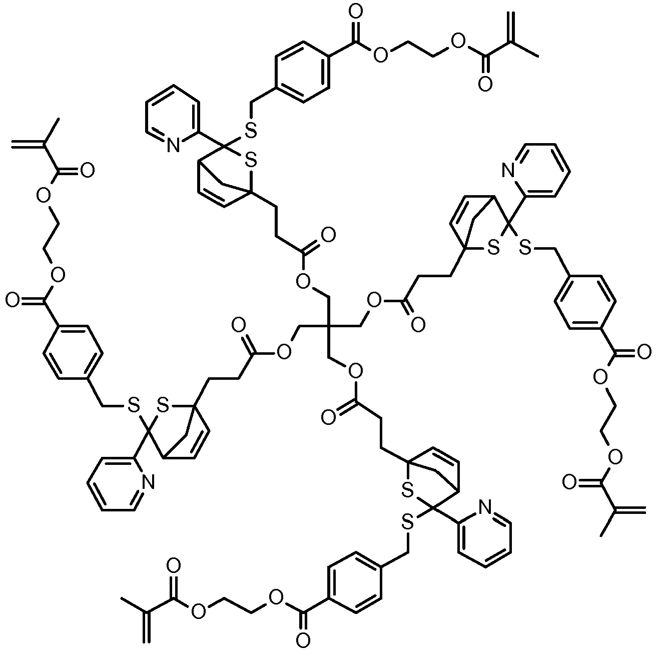

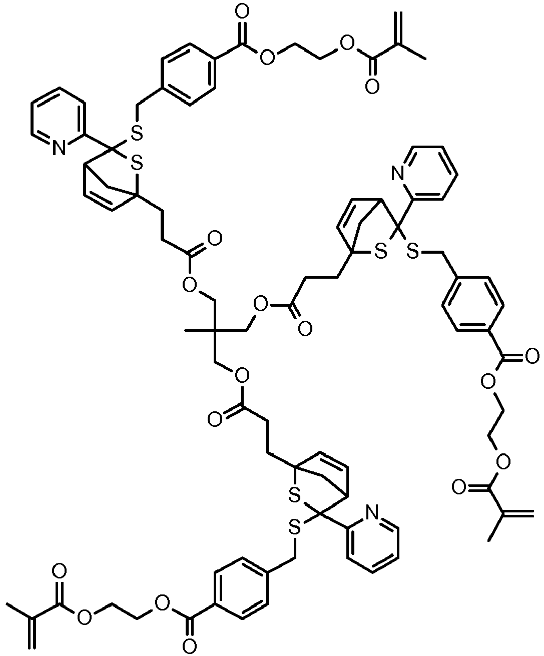

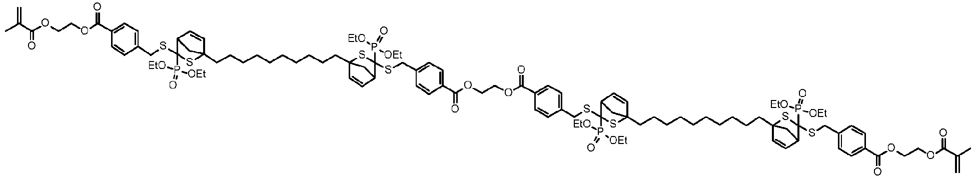

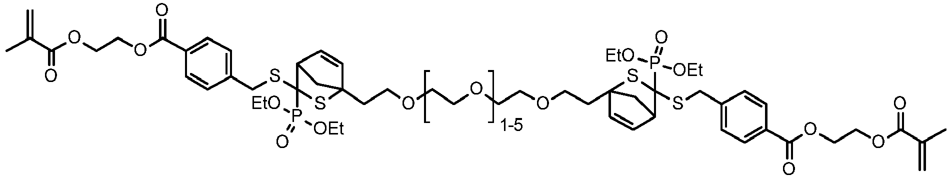

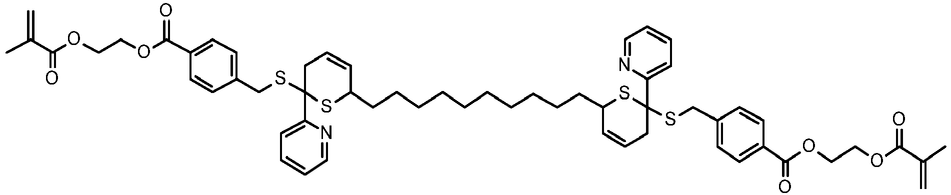

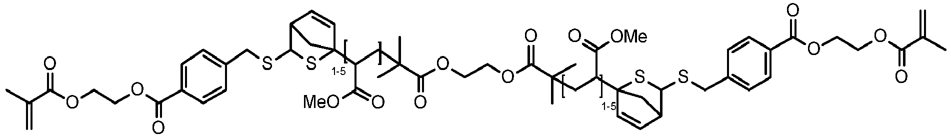

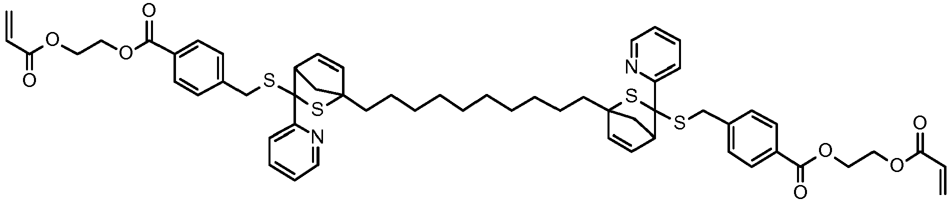

Die erfindungsgemässen Zusammensetzungen enthalten neben der thermolabilen polymerisierbaren Verbindung der Formel I vorzugsweise ein oder mehrere zusätzliche radikalisch polymerisierbare Monomere (Co-Monomere), insbesondere mono- oder polyfunktionelle (Meth)acrylsäurederivate. Unter monofunktionellen (Meth)acrylsäurederivaten werden Verbindungen mit einer, unter polyfunktionellen (Meth)acryl-säurederivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäss bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi(meth)acrylat, 1,4-Butandioldi(meth)acrylat, 1,10-Decan-dioldi(meth)acrylat und 1,12-Dodecandioldi(meth)acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)-methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis-(3-vinyl-2-pyrrolidonyl)-hexan, Bisacrylamide wie Methylen- oder Ethylenbisacrylamid und Bis(meth)acrylamide wie N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin, die durch Umsetzung aus den entsprechenden Diaminen mit (Meth)acrylsäurechlorid synthetisiert werden können.

Die erfindungsgemässen Zusammensetzungen können als Co-Monomere auch weitere thermolabile Vernetzermonomere enthalten. Thermolabile Vernetzermonomere weisen mindestens eine thermolabile Gruppe zwischen zwei polymerisierbaren Gruppen auf. Beispiele sind polyfunktionelle (Meth)acrylate oder (Meth)acrylamide mit mindestens einer thermolabilen Gruppe zwischen zwei (Meth)acrylgruppen. Dabei kommen als thermolabile Gruppen insbesondere thermolabile Alkoxyamin-, Oximester-, Oximurethan- oder Azogruppen, Peroxide und Disulfone in Frage. Beispiele sind die Umsetzungsprodukte von N-Hydroxy-(meth)acrylamid mit Di- oder Triisocyanaten wie Hexamethylen-1,6-diisocyanat (HDI), 2,2,4-Trimethylhexamethylen-1,6-diisocyanat oder dem HDI-Trimer sowie Produkte, die durch stöchiometrische Umsetzung von Di- oder Triisocyanaten mit 1-Hydroxymethylacrylsäureestern wie 1-Hydroxymethylacrylsäureethylester oder mit β-Ketoester(meth)acrylaten wie 2-Acetoacetoxyethylmethacrylat erhalten werden. Besonders geeignet sind auch gasfreisetzende thermolabile Vernetzermonomere. Beispiele sind die Veresterungsprodukte von Azobis(4-cyanovaleriansäure) mit Hydroxyalkyl(meth)acrylaten wie Hydroxyethyl(meth)acrylat oder Hydroxypropyl(meth)acrylat oder mit N-(Hydroxyalkyl)(meth)acrylamiden wie N-(5-Hydroxypentyl)methacrylamid oder N-Methyl-N-(2-hydroxyethyl)acrylamid.

Die erfindungsgemässen Zusammensetzungen können neben der thermolabilen polymerisierbaren Verbindung der Formel I und ggf. den oben genannten Co-Monomeren vorzugweise auch radikalkettenübertragungsaktive Additive enthalten, die die Struktur der Polymerisate, vor allem Molmasse, Molmassenverteilung, Netzwerkdichte, beeinflussen und die Einstellung von Eigenschaften wie Glasübergangstemperatur, Breite des Glasübergangs, Schlag- und Bruchzähigkeit ermöglichen. Bevorzugte radikalkettenübertragungsaktive Additive sind Dithioester, Trithiocarbonate, Allylsulfone, Vinylsulfonester oder andere bekannte RAFT-Reagenzien (RAFT = Reversible Addition-Fragmentation Chain Transfer).

Die erfindungsgemässen Zusammensetzungen können neben der thermolabilen polymerisierbaren Verbindung der Formel I und ggf. den oben genannten Co-Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisierbaren Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Monomere mit polymerisierbaren Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphon-säure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester.

Bevorzugte Monomere mit polymerisierbaren Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacryl-amido)hexyldihydrogenphosphat und 1,3-Bis-(N-acryloyl-N-propyl-amino)-propan-2-yl-dihydrogenphosphat.

Bevorzugte Monomere mit polymerisierbaren Sulfonsäuregruppen sind Vinylsulfonsäure, 4-Vinylphenylsulfonsäure und 3-(Methacrylamido)-propylsulfonsäure.

Vorzugsweise werden Mischungen der vorstehend genannten Monomere verwendet. Bevorzugte Monomermischungen enthalten bezogen auf das Gesamtgewicht der Monomermischung:
1 bis 90 Gew.-%, vorzugsweise 5 bis 80 Gew.-%, besonders bevorzugt 5 bis 70 Verbindung der Formel I,
0 bis 70 Gew.-%, vorzugsweise 1 bis 60 Gew.-%, besonders bevorzugt 5 bis 50 und ganz besonders bevorzugt 10 bis 30 Gew.-% Co-Monomer und insbesondere mono- und/oder polyfunktionelle (Meth)acrylate,
0 bis 70 Gew.-%, vorzugsweise 1 bis 60 Gew.-% und besonders bevorzugt 5 bis 50 Gew.-% weiteres thermolabiles Vernetzermonomer und 0 bis 40 Gew.-%, vorzugsweise 1 bis 30 Gew.-% und besonders bevorzugt 5 bis 20 Gew.-% Haftmonomer.

Besonders bevorzugte Monomermischungen (jeweils bezogen auf das Gesamtgewicht der Monomermischung) sind in der nachfolgenden Tabelle wiedergegeben:

| Komponente (Gew.-%) | A | B | C | D | E | F |
|---|---|---|---|---|---|---|
| Verbindung der Formel I | 1-90 | 5-80 | 5-70 | 5-70 | 5-70 | 5-70 |
| Co-Monomer, insbesondere mono- und/oder polyfunktionelles (Meth)acrylat | 0-70 | 0-60 | 1-60 | 5-60 | 5-50 | 0-30 |
| weiteres thermolabiles Vernetzermonomer | 0-70 | 0-50 | 0-50 | 5-50 | 5-50 | 5-50 |
| Haftmonomer | 0-40 | 0-30 | 0-30 | 0-20 | 0-20 | 0-30 |

Ausserdem enthalten die erfindungsgemässen Zusammensetzungen vorzugsweise auch einen Initiator für die radikalische Polymerisation.

Zur Initiierung der radikalischen Photopolymerisation insbesondere bei thermolabilen Verbindungen der Formel I werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4'-Dichlorbenzil eingesetzt. Besonders bevorzugt werden Campherchinon und 2,2-Dimethoxy-2-phenyl-acetophenon und ganz besonders bevorzugt α-Diketone in Kombination mit Aminen wie 4-(Dimethylamino)-benzoesäureester, N,N-Dimethylaminoethylmethacrylat, N,N-Dimethyl-sym.-xylidin oder Triethanolamin als Reduktionsmittel verwendet. Besonders geeignet sind auch Norrish-Typ-I-Photoinitiatoren, insbesondere Acyl- oder Bisacylphosphinoxide, Monoacyltrialkyl- oder Diacyldialkylgermanium-Verbindungen wie Benzoyltrimethylgermanium, Dibenzoyldiethylgermanium oder Bis-(4-methoxybenzoyl)diethylgermanium. Dabei lassen sich auch Mischungen der verschiedenen Photoinitiatoren wie beispielsweise Dibenzoyldiethylgermanium in Kombination mit Campherchinon und 4-Dimethylaminobenzoesäureethylester einsetzen.

Als Initiatoren für eine bei Raumtemperatur durchgeführte Polymerisation werden vorzugsweise Redox-Initiatorkombinationen wie beispielsweise Kombinationen von Benzoylperoxid mit N,N-Dimethyl-sym.-xylidin oder N,N-Dimethyl-p-toluidin verwendet. Darüber hinaus sind auch Redoxsysteme bestehend aus Peroxiden und solchen Reduktionsmitteln wie z.B. Ascorbinsäure, Barbituraten oder Sulfinsäuren besonders geeignet.

Die erfindungsgemässen Zusammensetzungen können zudem ein thermisch gasfreisetzendes Additiv enthalten. Geeignete gasfreisetzende Additive sind z.B. Azo-Verbindungen wie Azodicarbonamid, 2,2'-Azobisisobutyronitril oder 2,2'-Azobis(4-cyano-pentansäure), N-Nitrosoverbindungen, Hydrazide wie Benzolsulfonylhydrazid, Peroxide wie Dicumolperoxid oder Acetondicarbonsäure. Beispiele für solche Verbindungen sind etwa in St. Quinn, Plastics, Additives & Compounding 2001, 3, 16-21 beschrieben. Dabei kann die Zerfallstemperatur beispielsweise im Falle der Azoverbindungen in an sich bekannter Weise durch das Substituentenmuster eingestellt werden (vgl. D. Braun, R. Jakobi, Monatshefte Chemie 1982, 113, 1403-1414) .

Weiterhin können die erfindungsgemässen Zusammensetzungen ein Additiv enthalten, das eingestrahlte elektromagnetische Strahlung in Wärme umwandeln kann. Solche sogenannten Strahlung-Wärme-Umwandler sind organische, anorganische oder metallorganische Stoffe oder Hybridkomponenten, die in der Lage sind, UV-, NIR- oder IR-Strahlung, sichtbares Licht, Mikrowellen- oder Radiowellenstrahlung in Wärme umzuwandeln, um thermolabile Gruppen zu spalten. Beispiele hierfür sind UV-, NIR oder IR-Strahlung absorbierende Farbstoffe und Pigmente. Beispiele für im IR-Bereich absorbierende Farbstoffe sind Azo-, Methin-, Anthrachinon oder Porphyrinfarbstoffe. Beispiele für NIR-Strahlung absorbierende Pigmente sind Antimon- und Indiumzinnoxid, Phthalocyaninpigmente, Russ, Ni- und Pt-Dithiolen-Komplexe. Beispiele für im UV-Bereich absorbierende Verbindungen sind Benzotriazole, Triazine, Benzophenone, Cyanoacrylate, Salicylsäurederivate und Hindered Amine Light Stabilizers (HALS). Beispiele für Additive, die im Frequenzbereich der Mikrowellen (1 bis 300 GHz) oder Radiowellen (10 kHz bis 1 GHz) absorbieren, sind ferromagnetische keramische Stoffe, sogenannte Ferrite, die aus den Eisenoxiden Hämatit (Fe₂O₃) oder Magnetit (Fe₃O₄) und weiteren Oxiden beispielsweise der Metalle Zn, Mn, oder Ni aufgebaut und als Pulver kommerziell verfügbar sind.

Weiterhin enthalten die erfindungsgemässen Zusammensetzungen zur Verbesserung der mechanischen Eigenschaften oder zur Einstellung der Viskosität vorzugsweise auch organische oder anorganische Füllstoffpartikel. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgrösse von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgrösse von 5 bis 200 nm, vorzugsweise 10 bis 100 nm, Minifüllstoffe wie Quarz-, Glaskeramik- oder Glaspulver mit einer durchschnittlichen Teilchengrösse von 0,01 bis 10 µm, vorzugsweise 0,1 bis 1 µm, sowie röntgenopake Füllstoffe wie Ytterbiumtrifluorid oder nanopartikuläres Tantal (V)-oxid bzw. Bariumsulfat mit einer mittleren durchschnittlichen Partikelgrösse von 10 bis 1000 nm, vorzugsweise 100 bis 300 nm.

Ausserdem können die erfindungsgemässen Zusammensetzungen weitere Additive enthalten, vor allem Lösungsmittel wie Wasser oder Ethanol bzw. entsprechende Lösungsmittelgemische, sowie beispielsweise Stabilisatoren, Aromastoffe, Farbmittel, mikrobiozide Wirkstoffe, fluoridionenabgebende Additive, optische Aufheller, oder Weichmacher.

Besonders bevorzugt sind Zusammensetzungen auf Basis einer thermolabilen polymerisierbaren Verbindung der Formel I, welche die folgenden Bestandteile enthalten:
a) 0,1 bis 50 Gew.-%, insbesondere 1 bis 40 Gew.-%, bevorzugt 2 bis 30 Gew.-% und besonders bevorzugt 5 bis 30 Gew.-% Verbindung der Formel I,
b) 0,01 bis 10 Gew.-%, bevorzugt 0,1 bis 3,0 Gew.-% und besonders bevorzugt 0,2 bis 2 Gew.-% Initiator,
c) 0 bis 95 Gew.-%, bevorzugt 5 bis 95 Gew.-% und besonders bevorzugt 5 bis 90 Gew.-% Co-Monomer,
d) 0 bis 10 Gew.-%, insbesondere 0,1 bis 5 Gew.-% und bevorzugt 0,2 bis 3 Gew.-% Additiv.

Gefüllte Polymerisationsharze und Komposite auf der Basis der erfindungsgemässen Verbindungen der Formel I enthalten vorzugsweise die folgenden Bestandteile:
a) 0,1 bis 50 Gew.-%, insbesondere 1 bis 40 Gew.-% und bevorzugt 1 bis 20 Gew.-% Verbindung der Formel I,
b) 0,01 bis 10 Gew.-% und insbesondere 0,1 bis 3 Gew.-% Initiator,
c) 1 bis 80 Gew.-%, insbesondere 1 bis 60 Gew.-% und bevorzugt 5 bis 50 Gew.-% Co-Monomer,
d) 5 bis 90 Gew.-% und insbesondere 20 bis 90 Füllstoff,
e) 0 bis 10 Gew.-%, insbesondere 0,1 bis 5 Gew.-% und bevorzugt 0,2 bis 3 Gew.-% Additiv.

Dentalwerkstoffe auf der Basis der erfindungsgemässen Verbindungen der Formel I enthalten vorzugsweise die folgenden Bestandteile:
a) 0,1 bis 50 Gew.-%, insbesondere 1 bis 40 Gew.-%, bevorzugt 2 bis 30 Gew.-% und besonders bevorzugt 5 bis 30 Gew.-% Verbindung der Formel I,
b) 0,01 bis 10 Gew.-%, insbesondere 0,1 bis 3,0 Gew.-% und bevorzugt 0,2 bis 2 Gew.-% Initiator,
c) 0 bis 80 Gew.-%, insbesondere 1 bis 60 Gew.-% und bevorzugt 5 bis 50 Gew.-% Co-Monomer,
d) 0 bis 80 Gew.-% Füllstoff,
e) 0 bis 70 Gew.-% Lösungsmittel und
f) 0 bis 10 Gew.-%, insbesondere 0,1 bis 5 Gew.-% und bevorzugt 0,2 bis 3 Gew.-% Additiv.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff.

Dies gilt ebenso für den Lösungsmittelgehalt. Adhäsive enthalten vorzugweise bevorzugt 0 bis 60 Gew.-% und besonders bevorzugt 1 bis 50 Gew.-% Lösungsmittel. Zusammensetzungen, die Wasser als Lösungsmittel enthalten, sind bevorzugt. Besonders bevorzugt sind Zusammensetzungen, die 0 bis 20 Gew.-% und insbesondere 1 bis 10 Gew.-% Wasser enthalten.

Die Debonding-Eigenschaften von Zusammensetzungen auf der Basis der erfindungsgemässen Verbindungen der Formel I können gezielt durch die Zusammensetzung der Materialien beeinflusst werden. Die Einstellung einer für einen bestimmten Zweck geeigneten Zusammensetzung gehört zum allgemeinen Wissen und Können des Fachmanns. So nimmt mit der Konzentration an thermolabilen Komponenten, d.h. an den Verbindungen der Formel I sowie gegebenenfalls weiteren thermolabilen Vernetzern bzw. gasfreisetzenden Additiven im Adhäsiv oder Zement die Fähigkeit zum gezielten Debonding durch Erwärmen zu. Weiterhin lassen sich auch durch die Auswahl der Comonomeren die Debonding-Eigenschaften variieren. Dabei kann durch die Monomerauswahl der Glasübergangsbereich und damit der Erweichungsbereich der Polymerisate gezielt beeinflusst werden. Ausserdem lässt sich mit dem Anteil an vernetzenden Monomeren oder durch Zugabe von monofunktionellen Monomeren die Vernetzungsdichte und damit auch die Festigkeit und der E-Modul variieren.

Gegenstand der Erfindung ist auch die Verwendung einer erfindungsgemässen polymerisierbaren Zusammensetzung als Dentalwerkstoff oder zur Herstellung eines Dentalwerkstoffs, insbesondere eines Adhäsivs, Zements oder Füllungskomposits, sowie die Verwendung einer thermolabilen polymerisierbaren Verbindung der Formel I zur Herstellung von Dentalwerkstoffen, vorzugsweise Adhäsiven, Zementen oder Füllungskompositen und besonders bevorzugt selbstätzenden Adhäsiven, Zementen oder Füllungskompositen.

Die erfindungsgemässen Verbindungen der Formel I lassen sich auch zur Herstellung von Werkstoffen mit selbstheilenden Eigenschaften einsetzen. Dabei können durch gezieltes Tempern solcher Materialien die Polymernetzwerkstruktur verändert und Spannungen abgebaut oder Mikrorisse beseitigt werden. Weiterhin lassen sich die Verbindungen der Formel I zur Herstellung von stereolithographischen Formkörpern einsetzen. So können darauf basierende stereolithographisch hergestellte Keramikgrünköper ein verbessertes Entbinderungsverhalten zeigen. Im Falle von stereolithographisch gebauten Polymer- oder Kompositformkörpern aud Basis der erfindungsgemässen Verbindungen der Formel I lassen sich innere Spannungen durch eine Wärmebehandlung kontrolliert abbauen. Stereolithographisch hergestellte Wachsmodelle, die ein Polymernetzwerk auf Basis der erfindungsgemässen Verbindungen der Formel I enthalten, lassen sich einfach wieder entfernen, indem das Polymernetzwerk bei erhöhter Temperatur abgebaut wird und das Wachs dann ausfliessen kann.

Ferner lassen sich Verbindungen der Formel I zur Herstellung chemisch reversibler Montage- und Befestigungselemente, zur reversiblen Verankerung von Materialien oder Werkzeugen wie insbesondere Dübel, Anker, Schrauben, Beschläge oder sonstige Verbindungselemente in Mauerwerk, Beton, Leichtbau oder sonstigen Bauelementen einsetzen.

Gegenstand der Erfindung ist daher auch die Verwendung einer erfindungsgemässen polymerisierbaren Zusammensetzung als Polymerisationsharz, insbesondere als Adhäsiv, Komposit oder Stereolithographiewerkstoff, oder zur Herstellung eines Polymerisats, insbesondere eines Formteils, Duromers, Montage- oder Befestigungselements, sowie die Verwendung einer thermolabilen polymerisierbaren Verbindung der Formel I zur Herstellung eines Polymerisationsharzes oder Polymerisats und insbesondere zur Herstellung eines Adhäsivs, Komposits, Stereolithographiewerkstoffs, Formteils, Duromers oder Montage- oder Befestigungselements. Im Folgenden wird die Erfindung anhand von Ausführungsbeispielen näher erläutert.

### Ausführungsbeispiele

### Beispiel 1

### Synthese von Bis-1,10-(10-(3-(4-[2-(methacryloyloxy)-ethoxycarbonyl]-benzylsulfanyl]-3-pyridin-2-yl-2-thia-bicyclo[2.2.1]hept-5-en-1-ylmethyl)]-decan

### 1. Stufe: 2-Benzolsulfonylmethylpyridin

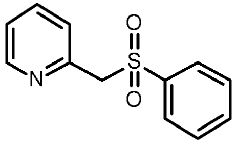

Eine Suspension von 2-(Chloromethyl)pyridinhydrochlorid (32,81 g, 0,20 mol) in Acetonitril (AN, 200 ml) wurde mit Natriumphenylsulfinat (49,24 g, 0,30 mol), Tetrapropylammoniumbromid (10,64 g, 40,0 mmol) und 1,8-Diazabicyclo[5.4.0]undec-7-en (DABCO, 30,44 g, 0,20 mol) versetzt. Das Reaktionsgemisch wurde 16 h am Rückfluss erhitzt und anschliessend am Rotationsverdampfer eingeengt. Der Rückstand wurde in Dichlormethan (DCM, 200 ml) aufgenommen, mit gesättigter wässriger NaCl-Lösung (3x 100 ml) gewaschen, über wasserfreiem Na₂SO₄ getrocknet, filtriert und am Rotationsverdampfer auf ca. das halbe Volumen eingeengt. Die braune Lösung wurde über eine Schicht Kieselgel filtriert (SiO₂, Ethylacetat). Man erhielt 41,55 g (89% Ausbeute) 2-Benzolsulfonylmethylpyridin als gelblichen Feststoff.
¹H-NMR (400 MHz, CDCl₃: *δ*, ppm) : 4,56 (s, 2H), 7,21-7,24 (m, 1H), 7,42-7,48 (m, 3H), 7,58-7,62 (m, 1H), 7,66-7,70 (m, 3H), 8,41-8,42 (m, 1H) .
¹³C-NMR (101 MHz, CDCl₃, *δ*, ppm): 64,6, 123,4, 125,7, 128,4, 129,0, 133,8, 136,7, 138,2, 148,8, 149,7.

### 2. Stufe: 4-(Pyridin-2-carbothioylsulfanylmethyl)-benzoesäure

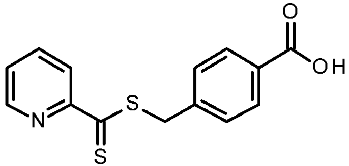

Zu einer Suspension von 2-Benzolsulfonylmethylpyridin (40,64 g, 0,174 mol) und Schwefel (16,76 g, 0,523 mol) in AN (500 ml) wurde unter Eiskühlung eine Lösung von DABCO (79.56 g, 0.523 mol) in AN (100 ml) zugetropft. Nach beendeter Zugabe wurde die dunkelrote Lösung 22 h bei Raumtemperatur (RT) gerührt, dann wurde 4-(Brommethyl)-benzoesäure (37,46 g, 0,174 mol) portionsweise zugegeben. Das Reaktionsgemisch wurde weitere 4 h bei Raumtemperatur gerührt und dann mit Salzsäure (2 N; 200 ml) versetzt (pH = 1). Die Suspension wurde filtriert. Der Filtrationsrückstand wurde mit AN (100 ml) gewaschen. Das Filtrat wurde mit Methyl-*tert*.-butylether (MtBE, 200 ml) und gesättigter wässriger NaCl-Lösung (100 ml) versetzt und die Phasen wurden getrennt. Die organische Phase wurde mit gesättigter wässriger NaCl-Lösung (2x 100 ml) gewaschen. Die vereinigten Wasserphasen wurden mit MtBE (100 ml) reextrahiert. Die vereinigten organischen Phasen wurden über wasserfreiem Na₂SO₄ getrocknet, filtriert und am Rotationsverdampfer eingeengt. Der Rückstand wurde mit dem oben erhaltenen Filtrationsrückstand vereinigt, mit AN (200 ml) versetzt und bei RT gerührt. Nach 4 h wurde die Suspension filtriert. Der Filtrationsrückstand wurde mit AN (50 ml) gewaschen und im Vakuumtrockenschrank getrocknet (50 °C, 125 mbar). Man erhielt 44,95 g (89% Ausbeute) 4-(Pyridin-2-carbothioylsulfanylmethyl)-benzoesäure als roten Feststoff.
¹H-NMR (400 MHz, CDCl₃: *δ*, ppm) : 4,66 (s, 2H) , 7,56-7,58 (m, 2H), 7,70-7,73 (m, 1H), 7,9 -7,96 (m, 2H), 7,00-8,03 (m, 1H), 8,26-8,28 (m, 1H), 8,66-8,68 (m, 1H), 13,03 (s, 1H).
¹³C-NMR (101 MHz, CDCl₃, *δ*, ppm): 39,6, 121,9, 127,8, 129,4, 129,5, 129,9, 137,7, 140,6, 148,3, 155,3, 166,9, 226,0.

### 3. Stufe: 4-(Pyridin-2-carbothioylsulfanylmethyl)-benzoesäure-2-(methacryloyloxy)-ethylester

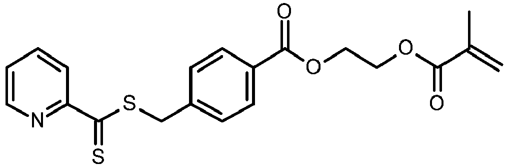

Eine Suspension von 4-(Pyridin-2-carbothioylsulfanylmethyl)-benzoesäure (10,39 g, 35,9 mmol), Hydroxyethylmethacrylat (4,67 g, 35,9 mmol) und *N,N*-Dimethylaminopyridin (DMAP, 0.60 g, 5,0 mmol) in DCM (100 ml) wurde auf 0 °C abgekühlt. 3-(Ethyliminomethylidenamino)-*N,N*-dimethyl-propan-1-aminhydrochlorid (EDC*HCl, 8,26 g, 43,1 mmol) wurde zugegeben und das Reaktionsgemisch wurde 1 h bei 0 °C und 16 h bei RT gerührt. Die rote Reaktionslösung wird über eine Schicht Kieselgel filtriert (SiO₂, DCM) und das Filtrat wurde am Rotationsverdampfer eingeengt. Der ölige rote Feststoff wurde mit n-Hexan (100 ml) versetzt, 20 h bei RT gerührt und filtriert. Der Filtrationsrückstand wurde mit n-Hexan (50 ml) gewaschen und im Vakuumtrockenschrank getrocknet (50 °C, 125 mbar). Man erhielt 11,22 g (78% Ausbeute) 4-(Pyridin-2-carbothioylsulfanylmethyl)-benzoesäure-2-(methacryloyloxy)-ethylester als hellroten Feststoff (Smp: 80-81 °C).
¹H-NMR (400 MHz, CDCl₃: *δ*, ppm) : 1,95 (s, 3H), 4,47-4,50 (m, 2H), 4,54-4,58 (m, 4H), 5,58-5,59 (m, 1H), 6,13-6,14 (m, 1H), 7,46-7,49 (m, 3H), 7,77-7,81 (m, 1H), 7,98-8,00 (m, 2H), 8,31-8,33 (m, 1H), 8,59-8,61 (m, 1H).
¹³C-NMR (101 MHz, CDCl₃, *δ*, ppm): 18,3, 40,8, 62,4, 62,7, 122,3, 126,2, 127,0, 129,0, 129,5, 130,0, 135,9, 137,0, 141,0, 148,0, 156,1, 166,0, 167,1, 225,4.

### 4. Stufe: 1,10-Bis(cyclopentadienyl)-decan (DiCp-decan)

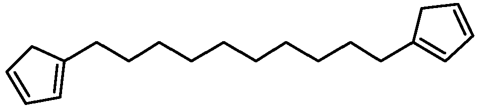

Zu einer Lösung von Dibromdecan (30,01 g, 0,10 mol) in wasserfreiem THF (400 ml) wurde bei -5 °C eine Natriumcyclopentadienid-Lösung (2.0 M in THF, 100,0 ml, 0,20 mol) zugetropft. Nach beendeter Zugabe wurde die trübe braune Lösung 1 h bei -5 °C und anschliessend bei RT weitergerührt. Nach 20 h wurde die Suspension über Kieselgel filtriert (SiO₂, Ethylacetat). Das Filtrat wurde am Rotationsverdampfer eingeengt. Der Rückstand wurde in n-Hexan (50 ml) gelöst, erneut über eine Schicht Kieselgel filtriert (SiO₂, n-Hexan) und das Filtrat wurde am Rotationsverdampfer eingeengt. Man erhielt 22,64 g (84% Ausbeute) des Isomerengemischs von 1,10-Bis(cyclopentadienyl)decan als farbloses Öl.
¹H-NMR (400 MHz, CDCl₃: *δ*, ppm): 1,22-1,34 (m, 12H), 1,46-1,58 (m, 4H), 2,29-2,42 (m, 4H), 2,79-2, 96 (m, 4H), 5,95-6, 46 (m, 6H) . ¹³C-NMR (101 MHz, CDCl₃, *δ*, ppm): 29,0, 29,6, 29,6, 29,7, 29,8, 29,9, 30,0, 30,8, 41,2, 43,3, 125,7, 126,2, 130,4, 132,5, 133,6, 134,9, 147,5, 150,2.

### 5. Stufe: Bis-1,10-{10-(3-{4-[2-(methacryloyloxy)-ethoxycarbonyl]-benzylsulfanyl}-3-pyridin-2-yl-2-thia-bicyclo[2.2.1]hept-5-en-1-ylmethyl) }-decan

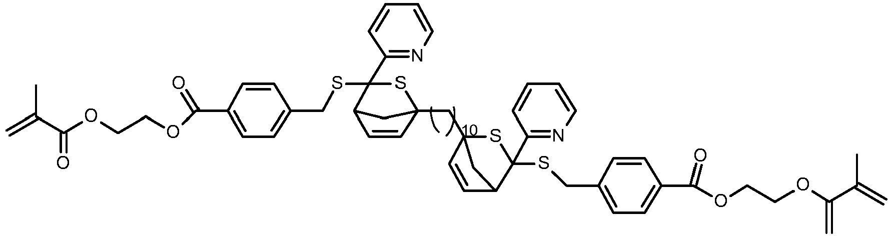

Eine Lösung von 4-(Pyridin-2-carbothioylsulfanylmethyl)-benzoesäure-2-(methacryloyloxy)-ethylester (16,06 g, 40,0 mmol) in Chloroform (80 ml) wurde mit Chlorwasserstoff (∼1.25 M in Methanol, 32,0 ml, 40,0 mmol) versetzt und 5 min bei RT gerührt. Eine Lösung von 1,10-Bis(cyclopentadienyl)-decan (5,41 g; 20,0 mmol) in Chloroform (20 ml) wurde zugetropft und das Reaktionsgemisch wurde bei RT gerührt. Nach 20 h wurde die Lösung mit Wasser (3x 50 ml) und gesättigter wässriger NaHCO₃-Lösung (50 ml) gewaschen, über wasserfreiem Na₂SO₄ getrocknet, filtriert und am Rotationsverdampfer eingeengt. Säulenchromatographische Reinigung (SiO₂, DCM/ Ethylacetat) ergab 16,82 g (78% Ausbeute) des Isomerengemischs von Bis-1,10-{10-(3-{4-[2-(methacryloyloxy)-ethoxycarbonyl]-benzyl-sulfanyl}-3-pyridin-2-yl-2-thia-bicyclo[2.2.1]hept-5-en-1-ylmethyl)}-decan als gelbliches Öl.
¹H-NMR (400 MHz, CDCl₃: *δ*, ppm) : 0,86-1,72 (m, 20H), 1,94 (s, 6H), 2,81-4,22 (m, 10H), 4,35-4,64 (m, 8H), 5,24-6,42 (m, 4H), 5,58 (s, 2H) , 6,13 (s, 2H), 6,93-8,57 (m, 16H).
¹³C-NMR (101 MHz, CDCl₃, *δ*, ppm): 18,3, 26,9, 28,7, 29,0, 29,1, 29,3, 29,5, 29,8, 29,9, 30,1, 33,5, 36,8, 37,2, 37,3, 49,7, 54,8, 56,5, 62,4, 62,5, 86,4, 121,4, 121,5, 124,9, 126,1, 126,2, 128,1, 128,9, 129,0, 129,0, 129,1, 129,6, 129,6, 135,9, 136,1, 136,3, 136,5, 143,7, 147,3, 152,9, 162,7, 166,1, 167,1.

### Beispiel 2

### Synthese von Bis (2-(methacryloyloxy) ethyl) 4,4'-(((1,1'-(decan-1,10-diyl)bis(3-(diethoxyphosphoryl)-2-thiabicyclo[2.2.1]hept-5-en-3,1-diyl))bis(sulfandiyl))bis(methylen))dibenzoat (DiHDA-Linker)

### 1. Stufe: 4-((((Diethoxyphosphoryl)carbonothioyl)thio)methyl)-benzoesäure (PDTMBA)

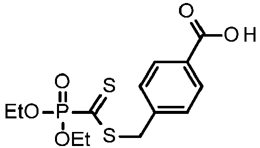

Zu einer Suspension von 1.5 g NaH (1.5 eq., 62 mmol) in 20 ml THF (trocken) wurde eine Lösung von 5.3 ml Diethylphosphit (1,0 eq., 41 mmol, 5,7 g) in wasserfreiem 40 ml THF bei RT zugetropft. Nach Beendigung der H₂-Entwicklung wurde 15 min zum Sieden erhitzt. Anschliessend wurde die Reaktionsmischung auf -90 °C abgekühlt und es wurden 12.3 ml Kohlenstoffdisulfid (CS₂, 5,0 eq., 205 mmol, 15,6 g) zugetropft. Es wurde 2 h bei RT gerührt, wobei sich das Reaktionsgemisch braun verfärbte. Es wurden 750 ml THF zugegeben. Anschliessend wurde eine Lösung von 10,0 g 4-Bromomethylbenzoesäure (1,1 eq., 46 mmol) in 75 ml THF langsam zugetropft und 16 h bei RT gerührt, wobei sich das Reaktionsgemisch violett verfärbte. Nach Entfernung des Lösungsmittels wurde der Rückstand in einer Mischung aus DCM und H₂O (1:1) aufgenommen. Die organische Phase wurde abgetrennt und die wässrige Phase mit DCM extrahiert. Die vereinigten organischen Phasen wurden über wasserfreiem Na₂SO₄ getrocknet und das Lösungsmittel wird entfernt. Das Produkt wurde anschliessend mittels Säulenchromatographie (Kieselgel 60, Cyclohexan:Ethylacetat:Essigsäure (1:1:0,01) gereinigt. Man erhielt 4.3 g (30% Ausbeute) PDTMBA als einen violetten Feststoff (Smp.: 113 °C).
¹H-NMR (400 MHz, CDCl₃: *δ*, ppm) : 1,37 (t, 6H, OCH₂C**H**₃), 4,22-4,34 (m, 4H, OC**H**₂CH₃), 4,54 (s, 2H, SCH₂), 7,41 (d, 2H, ArH), 8,05 (d, 2H, ArH), 10,50 (bs, 1H, COOH).
¹³C-NMR (101 MHz, CDCl₃, *δ*, ppm): 16,40 (OCH₂**C**H₃), 40,00 (SCH₂), 65,14 (O**C**H₂CH₃), 129,34 (Cₐᵣ), 129,53 (Cₐᵣ), 130,79 (Cₐᵣ), 140,04 (Cₐᵣ), 170,73 (COOH), 226,92 (PC=S), 228,66 (PC=S).

### 2. Stufe: 2-(Methacryloyloxy)ethyl-4-((((diethoxyphosphoryl)-carbono-thioyl)thio)methyl)benzoat (MA-PDTMBA))

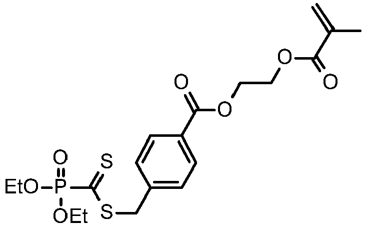

Zu einer Lösung von 2,0 g PDTMBA (1,0 eq., 5,74 mmol) und 1,05 ml 2-Hydroxyethylmethacrylat (HEMA, 1,5 eq., 8.61 mmol, 1,12 g) in DCM (trocken) wurde eine Lösung von 2,2 g EDC*HCl (2,0 eq., 11,5 mmol) und 0.14 g DMAP (0,2 eq., 1,15 mmol) in DCM (trocken) bei RT zugetropft. Nach 20 h Rühren wurde mit NaHCO₃-Lösung und Kochsalzlösung gewaschen. Anschliessend wurde über wasserfreiem Na₂SO₄ getrocknet und das Lösungsmittel entfernt. Das Produkt wurde mittels Säulenchromatographie (Kieselgel 60, Cyclohexan:EE (1:1)) gereinigt. Man erhielt 1.85 g (70% Ausbeute) an MA-PDTMBA als violette Flüssigkeit.
¹H-NMR (400 MHz, CDCl₃, *δ*, ppm) : 1,37 (t, 6H, OCH₂C**H**₃), 1,90 (s, 3H, CH₃C_{MA}), 4,22 - 4,34 (m, 4H, OC**H**₂CH₃), 4,42 - 4,58 (m, 4H, OC**H**₂C**H**₂O), 4,52 (s, 2H, SCH₂), 5,55 (d, 1H, CCH_{2,trans}), 6,10 (d, 1H, CCH_{2,cis}), 7,37 (d, 2H, ArH), 7,98 (d, 2H, ArH).
¹³C-NMR (101 MHz, CDCl₃, *δ*, ppm): 16,40 (OCH₂**C**H₃), 18,40 (C_{MA}**C**H₃), 40,00 (SCH₂), 62,60 (O**C**H₂**C**H₂O), 65,14 (O**C**H₂CH₃), 126,27 (**C**H₂C_{MA}), 129,48 (Cₐᵣ), 129,65 (Cₐᵣ), 130,28 (Cₐᵣ), 136,03 (C_{MA}), 139,50 (Cₐᵣ), 165,90 (Cₐᵣ**C**=O), 167,26 (C_{MA}**C**=O), 227,18 (PC=S), 228,93 (PC=S).

### 4. Stufe: Bis (2-(methacryloyloxy)ethyl)4,4'-(((1,1'-(decan-1,10-diyl)-bis(3-(diethoxyphosphoryl)-2-thiabicyclo[2.2.1]hept-5-en-3,1-diyl))bis(sulfandiyl))bis(methylen))dibenzoat (DiHDA-Linker)

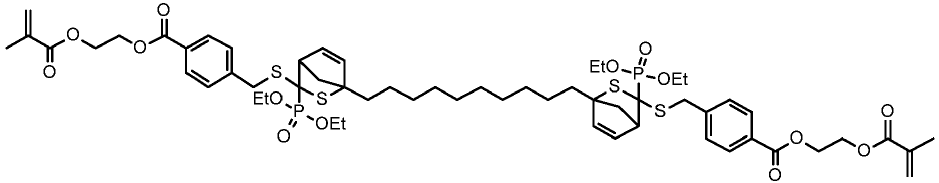

0,59 g 1,10-DiCp-decan (aus Beispiel 1, 4. Stufe, 1,0 eq., 2,17 mmol), 2,0 g MA-PDTMBA (2,0 eq., 4,34 mmol) und 30 mg ZnCl₂ (0,1 eq., 0,22 mmol) wurden in Ethylacetat gelöst und 30 min bei RT gerührt. Anschliessend wurde mit Wasser gewaschen und das Lösungsmittel entfernt. Man erhielt 2.56 g (99% Ausbeute) des DiHDA-Linkers als farblose hochviskose Flüssigkeit.
¹H-NMR (400 MHz, CDCl₃, *δ*, ppm): 1,25-1,65 (m, 28H, OCH₂C**H**₃, C_{HDA}CH₂(C**H**₂)₄), 1,94 (s, 6H, CH₃C_{MA}), 1,95-2,18 (m, 4H, CH_{2,HDA-Brücke}), 2,18-2,50 (m, 4H, C_{HDA}C**H**₂ (CH₂)₄), 3,35-3,68 (m, 2H, CH_{HDA}), 4,00-4,25 (m, 8H, OC**H**₂CH₃), 4,26-4,44 (m, 4H, SCH₂Cₐᵣ), 4,45-4,58 (m, 8H, OC**H**₂C**H**₂O), 5,52-5,60 (m, 1H, CH_{HDA-Db},), 5,58 (d, 2H, CCH_{2,trans}), 5,95-6,03 (2H, CH_{HDA-Db,}), 6,13 (d, 2H, CCH_{2,cis}), 6,28 - 6,33 (m, 1H, CH_{HDA-Db},), 7,42 (d, 4H, ArH), 7,95 (d, 4H, ArH).
MS: 1190,37 Da.

### Beispiel 3

### Herstellung eines Photopolymerisates des DiHDA-Linkers und spektroskopische Verfolgung der reversiblen thermischen Spaltung der HDA-Gruppen

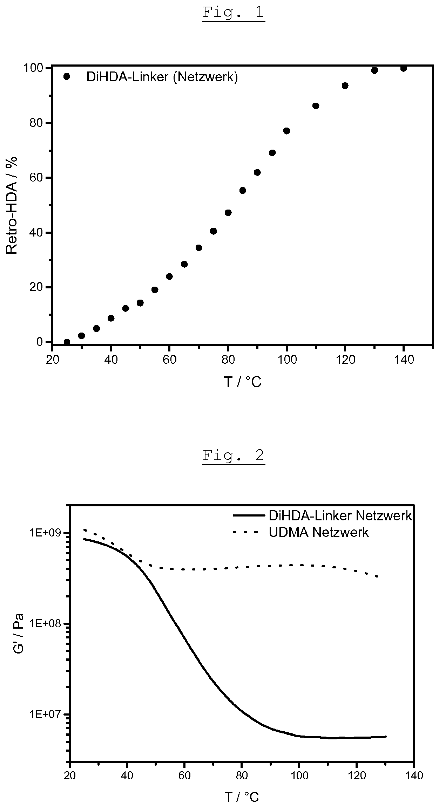

Der in Beispiel 2 hergestellte DiHDA-Linker wurde mit 0,2 Gew.-% Ivocerin (Bis-(4-methoxybenzoyl)-diethylgermanium) als Photoinitiator versetzt, in eine UV-Vis-Küvette mit einer Schichtdicke von 1 mm gefüllt und durch Bestrahlen (30 min) mit 3 Osram Dulux Blue Lampen (3x 18 W, > 200 mW/cm²) polymerisiert. Anschliessend wurden Absorptionsspektren im Bereich von 400-800 nm bei Temperaturen von 25 bis 140 °C des gebildeten Polymernetzwerkes mit einem Cary 300 Bio-Spektrometer mit einem temperaturregulierbaren Probenhalter aufgenommen. Der aus der Absorption im Maximum bei 530 nm im Temperaturbereich von 25 bis 140 °C berechnete Umsatz der Retro-Hetero-Diels-Alder-Reaktion (Retro-HDA-Reaktion) ist in Fig. 1 dargestellt.

Fig. 1 zeigt quantitativ an Hand der VIS-spektroskopisch detektierten Rückbildung der farbigen Dithioestereinheit durch die Retro-HDA-Reaktion das Aufbrechen des polymeren Netzwerkes in Abhängigkeit von der Temperatur. Hierbei steigt mit zunehmender Temperatur die Absorption im sichtbaren Bereich des Spektrums (Abs.ₘₐₓ: 530 nm) bis es bei 130 °C ein Maximum erreicht, welches den vollständigen Ablauf der Retro-HDA-Reaktion aufzeigt. Bei 25 °C kann keine Absorption im sichtbaren Bereich des Spektrums detektiert werden, was anzeigt, dass das Gleichgewicht der HDA-Reaktion bei dieser Temperatur vollständig auf der Seite der geschlossenen HDA-Form liegt. Dass bei Raumtemperatur nur die geschlossene Form vorliegt, konnte auch mittels ¹H-NMR-Spektroskopie des DiHDA-Linkers belegt werden. Auf Grund der Beobachtung, dass das Gleichgewicht der HDA-Reaktion bei 25 °C zu 0% und bei 130 °C zu 100% auf der Seite der Retro-HDA-Produkte liegt, kann für jede beliebige Temperatur in diesem Bereich der Grad an Retro-Reaktion angegeben werden und liegt die Halbwertstemperatur bei ca. 80 °C (vgl. Fig. 1).

### Beispiel 4

### Herstellung eines Photopolymerisates des DiHDA-Linkers und dessen dynamisch-mechanische Charakterisierung

Aus dem in Beispiel 2 hergestellte DiHDA-Linker wurden nach Zugabe von 0,2 Gew.-% Ivocerin als Photoinitiator Prüfkörper mit den Abmessungen L: 25 mm, B: 5 mm, D: 1 mm durch Bestrahlen (30 min) mit 3 Osram Dulux Blue Lampen (3x 18 W, > 200 mW/cm²) hergestellt. Als Referenzprobe wurden analoge Prüfkörper aus dem Urethandimethacrylat UDMA unter Zugabe von 0,2 Gew.-% Ivocerin als Photoinitiator und nachfolgender Bestrahlung hergestellt. Die Messung des Speichermoduls G' der hergestellten Proben erfolgte mittels ARES-G2 Rheometer (TA Instruments), das ein dehnungskontrolliertes Rotationsrheometer ist. Das Rheometer besitzt einen Kraftausgleichswandler, der Drehmomente zwischen 50 nN*m und 200 mN*m mit einer Genauigkeit von 1 nN*m ermöglicht. Die angelegte Oszillationsfrequenz kann zwischen 10⁻⁷ rad/s und 628 rad/s variiert werden. Für die Temperierung wurde ein Umluftofen mit Stickstoffzufuhr verwendet. Die temperaturabhängigen Messungen wurden mit einer Axialkraft von 0,3 N, einer Anregungsfrequenz von 1 Hz und einer Deformation von 0.1% in einem Temperaturbereich von 25 bis 130 °C (Heizrate: 1.5 K/min) durchgeführt und die G'-Ergebnisse für die Polymernetzwerke des DiHDA-Linkers und UDMA sind in Fig. 2 dargestellt. G' stellt den Fliesswiderstand einer Substanz dar und ist somit ein Mass für die Festigkeit eines Materials. Wie Fig. 2 dargestellt, zeigen das DiHDA-Linker-Polymernetzwerk und das UDMA-Netzwerk ein sehr unterschiedliches Verhalten mit steigender Temperatur. Der Speichermodul G' des UDMA-Netzwerkes zeigt im Bereich von 25 bis 130 °C nur einen leichten Abfall, wie es für nicht-thermoresponsive Netzwerke zu erwarten ist (1.1*10³ MPa bei 25 °C bis 3.8*10² MPa bei 120 °C). Dahingegen nimmt G' für das DiHDA-Linker-Netzwerk im gleichen Temperaturbereich drastisch von 8.5*10² MPa bei 25 °C auf 5.5 MPa bei 120 °C ab, was einer deutlichen Spaltung des Netzwerkes entspricht. Damit konnte gezeigt werden, dass auf der Basis der polymerisationsfähigen multifunktionellen Hetero-Diels-Alder-Addukte der allgemeinen Formel I Polymernetzwerke zugänglich sind, die sich bei Temperaturen deutlich unter 100 °C thermisch reversibel abbauen lassen.

### Beispiel 5

### Herstellung eines Photocopolymerisates des DiHDA-Linkers und dessen dynamisch-mechanische Charakterisierung

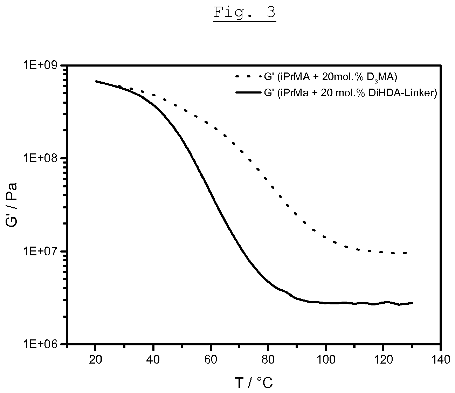

Der in Beispiel 2 hergestellte DiHDA-Linker (20 mol-%) wurde mit iso-Propylmethacrylat (iPMA) gemischt. Nach Zugabe von 0,2 Gew.-% Ivocerin als Photoinitiator wurden Prüfkörper mit den Abmessungen L: 25 mm, B: 5 mm, D: 1 mm durch Bestrahlen (30 min) mit 3 Osram Dulux Blue Lampen (3x 18 W, > 200 mW/cm²) hergestellt. Als Referenzprobe wurden analoge Prüfkörper aus einer Mischung des kommerziellen Vernetzers Decandioldimethacrylat (D₃MA) und iPMA (80 mol-%) unter Zugabe von 0,2 Gew.-% Ivocerin als Photoinitiator und nachfolgender Bestrahlung hergestellt. Die Messung des Speichermoduls G' erfolgte wie in Beispiel 4. Fig. 3 zeigt Vergleich des Speichermoduls G' eines Netzwerkes aus iPMA und dem Dimethacrylat D₃MA mit einem Netzwerk aus iPMA und dem DiHDA-Linker. Der Speichermodul G' des D₃MA-iPMA-Netzwerkes zeigt im Bereich von 25 bis 130 °C einen deutlich Abfall aufgrund des hohen Anteils des flexiblen Monomethacrylates iPMA. Dennoch nimmt G' für das DiHDA-Linker-iPMA-Netzwerk, das nur 20 mol-% an thermisch reversibel abbaubarem DiHDA-Linker enthält, im gleichen Temperaturbereich signifikant deutlicher ab.

### Beispiel 6

### Zementieren von Zahnkronen mit einem Peroxid-Amin-initiierten 2-Komponentenzement auf Basis des DiHDA-Linkers aus Beispiel 2

Aus Zirkoniumdioxid wurden Testabutments (Geometrie eines Kegelstumpfes, Durchmesser der Deckfläche: 4 mm, Durchmesser Grundfläche: 6 mm, Höhe: 3 mm) gefräst. Passend hierzu wurden Kronen derart aus Zirkoniumdioxid gefräst, dass durch das Aufliegen der Krone auf der Schulter des Testabutments ein Zementspalt von 0,2 mm resultiert. Kronen und Abutments wurden jeweils sandgestrahlt (110 µm Al₂O₃, 1 bar), in entionisiertem Wasser per Ultraschall für ca. 2 min gereinigt, getrocknet und anschliessend mit Monobond Plus (Ivoclar Vivadent AG, Verarbeitung gemäss Gebrauchsinformation: auftragen, für 60 s einwirken lassen, mit ölfreier Luft trockenblasen) für die Zementierung vorbereitet.

Für die Zementierung wurde ein selbsthärtendes Zweikomponentenzement (Mischungsverhältnis 1:1 w/w) enthaltend 20 mol% (bezogen auf die Monomermischung) des thermisch spaltbaren difunktionellen Vernetzers DiHDA aus Beispiel 2 (Zement A) angerührt. Dabei enthielt die erste Komponente 25,06 Gew.-% n-Butylmethacrylat (BuMA), 0,01 Gew.-% 2,6-Di-tert-butyl-p-kresol (BHT), 60,35 Gew.-% DiHDA-Linker und 14,58 Gew.-% BP-50-FT (50%-iges Dibenzoylperoxid, United Initiators) als Peroxid. Die zweite Komponente enthielt 33,33 Gew.-% BuMA, 63,67 Gew.-% DiHDA-Linker und 3 Gew.-% 3,5-Di-tert-butyl-N,N-diethylanilin (DABA). Die Mengen an BP-50-FT und DABA wurden dabei so gewählt, dass eine Verarbeitungszeit von ca. 1 bis 5 min resultierte.

Als Vergleich wurde in analoger Weise ein selbsthärtendes Zweikomponentenzement (Mischungsverhältnis 1:1 w/w) enthaltend 20 mol% (bezogen auf die Monomermischung) des nicht thermisch spaltbaren difunktionellen Vernetzers Bisphenol-A-glycidylmethacrylat (BisGMA) (Zement B) angerührt. Dabei enthielt die erste Komponente 47,90 Gew.-% BuMA, 47,10 Gew.-% BisGMA und 5 Gew.-% BP-50-FT. Die zweite Komponente enthielt 54,30 Gew.-% BuMA, 42,70 Gew.-% BisGMA und 3 Gew.-% DABA.

Jeweils zwei Tropfen des angemischten Zements wurden in eine vorbereitete Testkrone gegeben und das ebenfalls vorbereitete Testabutment aufgesetzt. Nach Belastung mit einem Gewicht von ca. 2 kg wurde der Zementüberschuss mit einem Wattepellet entfernt. Nach 10 min wurden Testkrone und Testabutment ausgespannt und für 3 Tage bei 23 ± 2 °C gelagert. Insgesamt wurden so 10 Kronen mit Zement A und 10 Kronen mit Zement B zementiert.

Zur Untersuchung der Ablösbarkeit wurden die jeweils 10 mit Zement A bzw. Zement B zementierten Prüfkörper zufällig in zwei gleichgrosse Gruppen à 5 Prüfkörper aufgeteilt und für 1 min in auf 23 °C (erste Gruppe) bzw. 80 °C (zweite Gruppe) temperiertes Wasser getaucht. Anschliessend wurden die Prüfkörper jeweils in einer Universalzugprüfmaschine (Zwick-Roell Z010) eingespannt, die Kronen mit einer konstanten Traversengeschwindigkeit von 1,0 mm/min vom Abutment abgezogen und die jeweils auftretende Maximalkraft als Abzugskraft registriert. Der Mittelwert der so ermittelten Abzugskraft ist jeweils in der nachfolgenden Tabelle dargestellt.

| Zement | Zement A (20 mol% DiHDA aus Beispiel 2; erfindungsgemäss) | Zement B (20 mol% Bis-GMA; Vergleich) |
|---|---|---|
| Abzugskraft [N] nach 1 min bei 23 °C | 663 ± 184 | 962 ± 229 |
| Abzugskraft [N] nach 1 min bei 80 °C | 42 ± 15 | 553 ± 171 |
| Prozentuale Verringerung der Abzugskraft durch Temperaturerhöhung von 23 °C auf 80 °C | 94 % | 42 % |

Diese Ergebnisse zeigen, dass nur mit dem erfindungsgemässen Zement A auf Basis des thermolabilen DiHDA-Linkers aus Beispiel 2 bereits nach einer Minute bei 80 °C ein praktisch vollständiges Debonding der zementierten Kronen erreicht wird.

## Patentansprüche

1. Polymerisierbare Zusammensetzung, die eine thermolabile polymerisierbare Verbindung der Formel I enthält:
A-[X-(T-X-Sp-X)ₚ-T-X-Q-(Z)ₙ]ₘ Formel I,
in der
A für einen m-wertigen linearen, verzweigten oder cyclischen aliphatischen oder aromatischen C₁-C₃₀-Rest, der durch -0-, -S-, -CO-O-, -O-CO-, -CO-NR³-, -NR³-CO-, -O-CO-NR³-, -NR³-CO-O- oder -NR³-CO-NR³- unterbrochen sein kann, oder eine oligomere Gruppe mit einer Molmasse von 200 bis 2000 g/mol steht,
Q jeweils unabhängig entfällt oder für einen (n+1)-wertigen linearen, verzweigten oder cyclischen aliphatischen oder aromatischen C₁-C₂₀-Rest steht, der durch -0-, -S-, -CO-O-, -O-CO-, -CO-NR³-, -NR³-CO-, -O-CO-NR³-, -NR³-CO-O- oder -NR3-CO-NR3- unterbrochen sein kann,
Sp jeweils unabhängig für einen linearen, verzweigten oder cyclischen aliphatischen oder aromatischen C₁-C₃₀-Rest, der durch -0-, -S-, -CO-O-, -O-CO-, -CO-NR³-, -NR³-CO-, -O-CO-NR³-, -NR³-CO-O- oder -NR³-CO-NR³ unterbrochen sein kann, oder eine oligomere Gruppe mit einer Molmasse von 200 bis 2000 g/mol steht,
T jeweils unabhängig ausgewählt ist aus 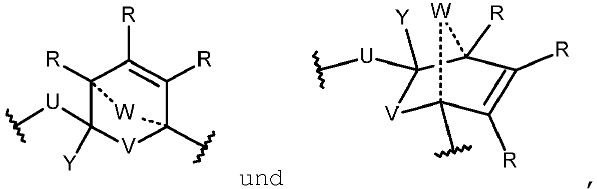
R jeweils unabhängig für H oder einen C₁-C₇-Alkyl-Rest steht,
U für S steht,
V für S steht,
W jeweils unabhängig entfällt oder für CH₂, O, S oder NH steht,
X jeweils unabhängig entfällt oder für -0-, -S-, -CO-O-, -O-CO-, -CO-NR³-, -NR³-CO-, -O-CO-NR³-, -NR³-CO-O- oder -NR³-CO-NR³- steht,
Y jeweils unabhängig für H, R⁴, Aryl, Heteroaryl, -CN, -OR⁴, -N(R⁴)₂ oder -P(O)(OR⁴)₂ steht,
Z jeweils unabhängig für eine polymerisierbare Gruppe ausgewählt aus Vinylgruppen, CH₂=CR-CO-O- und CH₂=CR¹-CO-NR²- steht,
R¹, R² und R³ jeweils unabhängig für H oder einen linearen oder verzweigten C₁-C₉-Alkyl-Rest stehen,
R⁴ jeweils unabhängig für einen linearen oder verzweigten C₁-C₉-Alkyl-Rest steht,
m die Werte 2 bis 6 annehmen kann,
n jeweils unabhängig die Werte 1, 2 oder 3 annehmen kann und
p die Werte 0 bis 10 annehmen kann.

2. Zusammensetzung nach Anspruch 1, bei der
A für einen m-wertigen linearen oder verzweigten aliphatischen oder aromatischen C₂-C₂₀-Rest, insbesondere C₄-C₁₈-Rest, bevorzugt C₈-C₁₆-Rest und besonders bevorzugt C₁₀-C₁₄-Rest, der durch -0-, -CO-O-, -O-CO-, -CO-NR³-,-NR³-CO-, -O-CO-NR³- oder -NR³-CO-O- unterbrochen sein kann, oder eine oligomere Gruppe mit einer Molmasse von 400 bis 1000 g/mol steht,
Q jeweils unabhängig entfällt oder für einen (n+1)-wertigen linearen oder verzweigten aliphatischen oder aromatischen C₂-C₁₅-Rest, insbesondere C₃-C₁₂-Rest und bevorzugt C₅-C₁₀-Rest steht, der durch -0-, -CO-O-,-O-CO-, -CO-NR³-, -NR³-CO-, -O-CO-NR²- oder -NR³-CO-O-unterbrochen sein kann, und besonders bevorzugt für-CH₂-Phenylen-CO-O-CH₂-CH₂- steht,
Sp jeweils unabhängig für einen linearen oder verzweigten aliphatischen oder aromatischen C₂-C₂₀-Rest, insbesondere C₄-C₁₈-Rest, bevorzugt C₈-C₁₆-Rest und besonders bevorzugt C₁₀-C₁₄-Rest, der durch -O-, -CO-O-, -O-CO-,-CO-NR³-, -NR³-CO-, -O-CO-NR³- oder -NR³-CO-O-unterbrochen sein kann, oder eine oligomere Gruppe mit einer Molmasse von 400 bis 1000 g/mol steht,
X jeweils unabhängig entfällt oder für -0-, -CO-O-, -O-CO-, -CO-NR³-, -NR³-CO-, -O-CO-NR³⁻ oder -NR²-CO-O- steht,
Z jeweils unabhängig für eine polymerisierbare Gruppe ausgewählt aus CH₂=CR¹-CO-O- und CH₂=CR¹-CO-NR²- steht,
R¹, R² und R³ jeweils unabhängig für H oder einen C₁-C₇-Alkyl-Rest, insbesondere H oder einen C₁-C₅-Alkyl-Rest und bevorzugt H oder einen C₁-C₃-Alkyl-Rest stehen,
R⁴ jeweils unabhängig für einen C₁-C₇-Alkyl-Rest, insbesondere einen C₁-C₅-Alkyl-Rest, bevorzugt einen C₁-C₃-Alkyl-Rest und besonders bevorzugt für Methyl, Ethyl oder Isopropyl steht,
m die Werte 2, 3 oder 4 annehmen kann und bevorzugt 2 ist,
n jeweils unabhängig die Werte 1 oder 2 annehmen kann und bevorzugt 1 ist und
p die Werte 0, 1, 2 oder 3 annehmen kann, insbesondere 0 oder 1 ist und bevorzugt 0 ist.

3. Zusammensetzung nach einem der Ansprüche 1 bis 2, bei der
R jeweils unabhängig für H oder einen C₁-C₅-Alkyl-Rest steht.

4. Zusammensetzung nach einem der Ansprüche 1 bis 3, bei der
W entfällt oder für CH₂ steht und
Y jeweils unabhängig für Heteroaryl oder -P(O)(OR⁴)₂ steht.

5. Zusammensetzung nach Anspruch 4, bei der
Y jeweils unabhängig für Pyridyl oder -P(O)(OEt)₂ steht.

6. Zusammensetzung nach einem der vorhergehenden Ansprüche, die ein oder mehrere zusätzliche radikalisch polymerisierbare Monomere enthält, ausgewählt aus:
Methyl-, Ethyl-, Hydroxyethyl-, Butyl-, Benzyl-, Tetrahydrofurfuryl- oder Isobornyl(meth)acrylat, Bisphenol-A-di(meth)-acrylat, Bis-GMA, UDMA, Di-, Tri- oder Tetraethylenglycoldi(meth)acrylat, Trimethylolpropantri-(meth)acrylat, Pentaerythrittetra(meth)acrylat, Glycerindi-(meth)acrylat, 1,4-Butandioldi(meth)acrylat, 1,10-Decandioldi(meth)acrylat, 1,12-Dodecandioldi (meth) acrylat,
und/oder
ein oder mehrere N-mono- oder -disubstitiuierte Acrylamide, N-Ethylacrylamid, N,N-Dimethacrylamid, N-(2-Hydroxyethyl)-acrylamid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether,
und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acryl-amido)-propan, 1,3-Bis-(methacrylamido)-propan, 1,4-Bis-(acryl-amido)-butan, 1,4-Bis(acryloyl)-piperazin,
oder eine Mischung davon.

7. Zusammensetzung nach einem der vorhergehenden Ansprüche, die ein oder mehrere radikalisch polymerisierbare, säuregruppenhaltige Monomere enthält, ausgewählt aus:
Maleinsäure, Acrylsäure, Methacrylsäure, 2-(Hydroxymethyl)-acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin, 4-Vinylbenzoesäure,
und/oder
Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure, 2-[4-(Dihydroxyphosphoryl)-2-oxa-butyl]-acrylsäureethyl-oder -2,4,6-trimethylphenylester,
und/oder
2-Methacryloyloxypropylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogen-phosphat, 1,3-Bis-(N-acryloyl-N-propyl-amino)-propan-2-yldi-hydrogenphosphat,
und/oder
Vinylsulfonsäure, 4-Vinylphenylsulfonsäure, 3-(Methacrylamido)-propylsulfonsäure,
oder eine Mischung davon.

8. Zusammensetzung nach einem der vorhergehenden Ansprüche, die einen Initiator für die radikalische Polymerisation enthält.

9. Zusammensetzung nach einem der vorhergehenden Ansprüche, die ein thermisch gasfreisetzendes Additiv enthält.

10. Zusammensetzung nach einem der vorhergehenden Ansprüche, die ein Additiv enthält, das eingestrahlte elektromagnetische Strahlung in Wärme umwandeln kann.

11. Zusammensetzung nach einem der vorhergehenden Ansprüche, die organischen und/oder anorganischen Füllstoff enthält.

12. Zusammensetzung nach einem der vorhergehenden Ansprüche, die
a) 0,1 bis 50 Gew.-% Verbindung der Formel I,
b) 0,01 bis 10 Gew.-% Initiator,
c) 0 bis 80 Gew.-% Co-Monomer,
d) 0 bis 80 Gew.-% Füllstoff,
e) 0 bis 70 Gew.-% Lösungsmittel und
f) 0 bis 10 Gew.-% Additiv enthält.

13. Zusammensetzung nach Anspruch 11 oder 12, die 0 bis 20 Gew.-% Füllstoff enthält.

14. Zusammensetzung nach Anspruch 11 oder 12, die 20 bis 80 Gew.-% Füllstoff enthält.

15. Zusammensetzung nach einem der Ansprüche 1 bis 14 zur Verwendung als Dentalwerkstoff.

16. Verwendung einer Zusammensetzung nach einem der Ansprüche 1 bis 14 als Adhäsiv, Komposit oder Stereolithographiewerkstoff, oder zur Herstellung eines Formteils, Duromers, Montage- oder Befestigungselements.

17. Verwendung einer Verbindung der Formel I wie in einem der Ansprüche 1 bis 5 definiert zur Herstellung eines Dentalwerkstoffs.

18. Verwendung einer Verbindung der Formel I wie in einem der Ansprüche 1 bis 3 definiert zur Herstellung eines Adhäsivs, Komposits, Stereolithographiewerkstoffs, Formteils, Duromers oder Montage- oder Befestigungselements.

## Claims

1. Polymerisable composition, which comprises a thermolabile polymerisable compound of Formula I:
A-[X-(T-X-Sp-X)ₚ-T-X-Q-(Z)ₙ]ₘ Formula I,
in which
A represents an m-valent linear, branched or cyclic aliphatic or aromatic C₁-C₃₀ group which can be interrupted by -O-, -S-, -CO-O-, -O-CO-, -CO-NR³-, -NR³-CO-, -O-CO-NR³-, -NR³-CO-O- or -NR³-CO-NR³-, or represents an oligomeric group with a molecular weight of from 200 to 2000 g/mol,
Q in each case independently is absent or represents an (n+1)-valent linear, branched or cyclic aliphatic or aromatic C₁-C₂₀ group which can be interrupted by -O-, -S-, -CO-O-, -O-CO-, -CO-NR³-, -NR³-CO-, -O-CO-NR³-, -NR³-CO-O-or -NR³-CO-NR³-,
Sp in each case independently represents a linear, branched or cyclic aliphatic or aromatic C₁-C₃₀ group which can be interrupted by -O-, -S-, -CO-O-, -O-CO-,-CO-NR³-, -NR³-CO-, -O-CO-NR³-, -NR³-CO-O- or -NR³-CO-NR³-, or represents an oligomeric group with a molecular weight of from 200 to 2000 g/mol,
T is selected in each case independently from 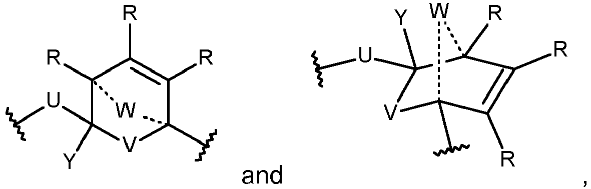
R in each case represents independently H or a C₁-C₇ alkyl group,
U stands for S,
V stands for S,
W in each case independently is absent or represents CH₂, O, S or NH,
X in each case independently is absent or represents -O-, -S-, -CO-O-, -O-CO-, -CO-NR³-, -NR³-CO-, -O-CO-NR³-, -NR³-CO-O- or -NR³-CO-NR³-,
Y in each case independently represents H, R⁴, aryl, heteroaryl, -CN, -OR⁴,-N(R⁴)₂ or -P(O)(OR⁴)₂,
Z in each case independently represents a polymerisable group selected from vinyl groups, CH₂=CR¹-CO-O- and CH₂=CR¹-CO-NR²-,
R¹, R² and R³ in each case independently represent H or a linear or branched C₁-C₉ alkyl group,
R⁴ in each case independently represents a linear or branched C₁-C₉ alkyl group,
m can assume the values 2 to 6,
n in each case independently can assume the values 1, 2 or 3 and
p can assume the values 0 to 10.

2. Composition according to claim 1, in which
A represents an m-valent linear or branched aliphatic or aromatic C₂-C₂₀ group, in particular a C₄-C₁₈ group, preferably a C₈-C₁₆ group and particularly preferably a C₁₀-C₁₄ group, which can be interrupted by -O-, -CO-O-, -O-CO-, -CO-NR³-, -NR³-CO-, -O-CO-NR³- or -NR³-CO-O-, or represents an oligomeric group with a molecular weight of from 400 to 1000 g/mol,
Q in each case independently is absent or represents an (n+1)-valent linear or branched aliphatic or aromatic C₂-C₁₅ group, in particular a C₃-C₁₂ group and preferably a C₅-C₁₀ group, which can be interrupted by-O-, -CO-O-, -O-CO-, -CO-NR³-, -NR³-CO-, -O-CO-NR³- or -NR³-CO-O-, and particularly preferably represents -CH₂-pheny|ene-CO-O-CH₂-CH₂-,
Sp in each case independently represents a linear or branched aliphatic or aromatic C₂-C₂₀ group, in particular a C₄-C₁₈ group, preferably a C₈-C₁₆ group and particularly preferably a C₁₀-C₁₄ group, which can be interrupted by -O-, -CO-O-, -O-CO-, -CO-NR³-, -NR³-CO-, -O-CO-NR³- or-NR³-CO-O-, or represents an oligomeric group with a molecular weight of from 400 to 1000 g/mol,
X in each case independently is absent or represents -O-, -CO-O-, -O-CO-, -CO-NR³-, -NR³-CO-, -O-CO-NR³- or -NR³-CO-O,
Z in each case independently represents a polymerisable group selected from CH₂=CR¹-CO-O- and CH₂=CR¹-CO-NR²-,
R¹, R² and R³ in each case independently represent H or a C₁-C₇ alkyl group, in particular H or a C₁-C₅ alkyl group and preferably H or a C₁-C₃ alkyl group,
R⁴ in each case independently represents a C₁-C₇ alkyl group, in particular a C₁-C₅ alkyl group, preferably a C₁-C₃ alkyl group and particularly preferably represents methyl, ethyl or isopropyl,
m can assume the values 2, 3 or 4 and is preferably 2,
n in each case independently can assume the values 1 or 2 and is preferably 1 and
p can assume the values 0, 1, 2 or 3, in particular 0 or 1, and is preferably 0.

3. Composition according to one of claims 1 to 2, in which
R In each case represents independently H or a C₁-C₅ alkyl group,

4. Composition according to one of claims 1 to 3, in which
W Is absent or represents CH₂ and
Y in each case independently represents heteroaryl or -P(O)(OR⁴)₂.

5. Composition according to claim 4, in which
Y in each case independently represents pyridyl or -P(O)(OEt)₂.

6. Composition according to one of the preceding claims, which comprises one or more additional radically polymerisable monomers, selected from:
methyl, ethyl, hydroxyethyl, butyl, benzyl, tetrahydrofurfuryl or *iso*bornyl (meth)acrylate, bisphenol A di(meth)acrylate, bis-GMA, UDMA, di-, tri- or tetraethylene glycol di(meth)acrylate, trimethylolpropane tri(meth)acrylate, pentaerythritol tetra(meth)acrylate, glycerol di(meth)acrylate, 1,4-butanediol di(meth)acrylate, 1,10-decanediol di(meth)acrylate, 1,12-dodecanediol di(meth)acrylate,
and/or
one or more *N*-mono- or *N*-disubstituted acrylamides, *N*-ethylacrylamide, N,N-dimethacrylamide, *N*-(2-hydroxyethyl)acrylamide, *N*-methyl-*N*-(2-hydroxyethyl)acrylamide, one or more *N*-monosubstituted methacrylamides, N-ethylmethacrylamide, *N*-(2-hydroxyethyl)methacrylamide, *N*-vinyl pyrrolidone, one or more cross-linking allyl ethers,
and/or
one or more cross-linking pyrrolidones, 1,6-bis(3-vinyl-2-pyrrolidonyl)-hexane, one or more cross-linking bisacrylamides, methylene or ethylene bisacrylamide, one or more cross-linking bis(meth)acrylamides, *N,N*'-diethyl-1,3-bis(acrylamido)propane, 1,3-bis(methacrylamido)propane, 1,4-bis(acrylamido)butane, 1,4-bis(acryloyl)piperazine,
or a mixture thereof.

7. Composition according to one of the preceding claims, which comprises one or more radically polymerisable, acid group-containing monomers, selected from:
maleic acid, acrylic acid, methacrylic acid, 2-(hydroxymethyl)acrylic acid, 4-(meth)acryloyloxyethyltrimellitic anhydride, 10-methacryloyloxydecylmalonic acid, N-(2-hydroxy-3-methacryloyloxypropyl)-*N*-phenylglycine, 4-vinylbenzoic acid,
and/or
vinylphosphonic acid, 4-vinylphenylphosphonic acid, 4-vinylbenzylphosphonic acid, 2-methacryloyloxyethylphosphonic acid, 2-methacrylamidoethylphosphonic acid, 4-methacrylamido-4-methylpentylphosphonic acid, 2-[4-(dihydroxyphosphoryl)-2-oxa-butyl]-acrylic acid, the ethyl ester or 2,4,6-trimethylphenyl ester of 2-[4-(dihydroxyphosphoryl)-2-oxabutyl]-acrylic acid,
and/or
2-methacryloyloxypropyl mono- or dihydrogen phosphate, 2-methacryloyloxyethylphenyl hydrogen phosphate, dipentaerythritolpentamethacryloyloxyphosphate, 10-methacryloyloxydecyl dihydrogen phosphate, mono-(1-acryloyl-piperidin-4-yl) phosphate, 6-(methacrylamido)hexyl dihydrogen phosphate, 1,3-bis-(*N-*acryloyl-*N*-propyl-amino)-propan-2-yl dihydrogen phosphate,
and/or
vinylsulfonic acid, 4-vinylphenylsulfonic acid, 3-(methacrylamido)propylsulfonic acid,
or a mixture thereof.

8. Composition according to one of the preceding claims, comprising an initiator for the radical polymerisation.

9. Composition according to one of the preceding claims, comprising an additive that when heated releases gas.

10. Composition according to one of the preceding claims, comprising an additive that can convert radiated electromagnetic radiation into heat.

11. Composition according to one of the preceding claims, comprising organic and/or inorganic filler.

12. Composition according to one of the preceding claims, comprising
a) 0.1 to 50 wt % of the compound of Formula I,
b) 0.01 to 10 wt % initiator,
c) 0 to 80 wt % comonomer,
d) 0 to 80 wt % filler,
e) 0 to 70 wt % solvent and
f) 0 to 10 wt % additive.

13. Composition according to claim 11 or 12, comprising 0 to 20 wt % filler.

14. Composition according to claim 11 or 12, comprising 20 to 80 wt % filler.

15. Composition according to one of claims 1 to 14 for use as a dental material.

16. Use of a composition according to one of claims 1 to 14 as an adhesive, composite or stereolithographic material, or for preparing a moulded object, thermoset, mounting or fixing element.

17. Use of a compound of Formula I as defined in one of claims 1 to 5 for preparing a dental material.

18. Use of a compound of Formula I as defined in one of claims 1 to 3 for preparing an adhesive, composite, stereolithographic material, moulded object, thermoset or mounting or fixing element.

## Revendications

1. Composition polymérisable, qui contient un composé polymérisable thermolabile de formule I :
Formule I : A-[X-(T-X-Sp-X)ₚ-T-X-Q-(Z)ₙ]ₘ
dans laquelle
A représente un groupe en C₁-C₃₀, de valence m, aliphatique linéaire, ramifié ou cyclique, ou aromatique, qui peut être interrompu par un ou des chaînons symbolisé(s) par -O-, -S-, -CO-O-, -O-CO-, -CO-NR³-, -NR³-CO-, -O-CO-NR³-, -NR³-CO-O- ou -NR³-CO-NR³-, ou encore un groupe oligomère présentant une masse molaire de 200 à 2000 g/mol,
Q indépendamment en chaque occurrence, ne représente rien ou représente un groupe en C₁-C₂₀, de valence n+1, aliphatique linéaire, ramifié ou cyclique, ou aromatique, qui peut être inter-rompu par un ou des chaînons symbolisé(s) par -O-, -S-, -CO-O-, -O-CO-, -CO-NR³-, -NR³-CO-, -O-CO-NR³-, -NR³-CO-O-ou -NR³-CO-NR³-,
Sp indépendamment en chaque occurrence, représente un groupe en C₁-C₃₀, aliphatique linéaire, ramifié ou cyclique, ou aromatique, qui peut être interrompu par un ou des chaînons symbolisé(s) par -O-, -S-, -CO-O-, -O-CO-, -CO-NR³-, -NR³-CO-, -O-CO-NR³-, -NR³-CO-O- ou -NR³-CO-NR³-, ou encore un groupe oligomère présentant une masse molaire de 200 à 2000 g/mol,
T indépendamment en chaque occurrence, représente un groupe choisi parmi ceux de formules 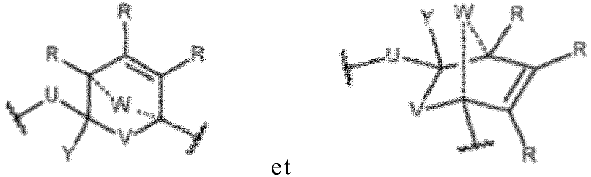
R indépendamment en chaque occurrence, représente un atome d'hydrogène ou un groupe alkyle en C₁-C₇,
U représente un atome de soufre,
V représente un atome de soufre,
W indépendamment en chaque occurrence, ne représente rien ou représente un chaînon symbolisé par CH₂, O, S ou NH,
X indépendamment en chaque occurrence, ne représente rien ou représente un ou des chaînons symbolisé(s) par -O-, -S-, -CO-O-, -O-CO-, -CO-NR³-, -NR³-CO-, -O-CO-NR³-, -NR³-CO-O-ou -NR³-CO-NR³-,
Y indépendamment en chaque occurrence, représente un atome d'hydrogène, une entité symbolisée par R⁴, un groupe aryle ou hétéroaryle, un groupe cyano, ou une entité symbolisée par -OR⁴, -N(R⁴)₂, ou -P(O)(OR⁴)₂,
Z indépendamment en chaque occurrence, représente un groupe polymérisable choisi parmi le groupe vinyle et les groupes de formule CH₂=CR¹-CO-O- ou CH₂=CR¹-CO-NR²-,
R¹, R² et R³ représentent chacun, indépendamment en chaque occurrence, un atome d'hydrogène ou un groupe alkyle en C₁-C₉, linéaire ou ramifié,
R⁴ indépendamment en chaque occurrence, représente un groupe alkyle en C₁-C₉, linéaire ou ramifié,
l'indice m peut valoir de 2 à 6,
l'indice n, indépendamment en chaque occurrence, peut valoir 1, 2 ou 3,
et l'indice p peut valoir de 0 à 10.

2. Composition conforme à la revendication 1, dans laquelle
A représente un groupe de valence m, aliphatique linéaire ou ramifié ou aromatique, en C₂-C₂₀, en particulier en C₄-C₁₈, de préférence en C₈-C₁₆ et mieux encore en C₁₀-C₁₄, qui peut être interrompu par un ou des chaînons symbolisé(s) par -O-, -CO-O-, -O-CO-, -CO-NR³-, -NR³-CO-, -O-CO-NR³- ou -NR³-CO-O-, ou encore un groupe oligomère présentant une masse molaire de 400 à 1000 g/mol,
Q indépendamment en chaque occurrence, ne représente rien ou représente un groupe de valence n+1, aliphatique linéaire ou ramifié, ou aromatique, en C₂-C₁₅, en particulier en C₃-C₁₂ et de préférence en C₅-C₁₀, qui peut être interrompu par un ou des chaînons symbolisé(s) par -O-, -CO-O-, -O-CO-, -CO-NR³-, -NR³-CO-, -O-CO-NR³- ou -NR³-CO-O, mais représente, avec une préférence particulière, un groupe -CH₂-phénylène-CO-O-CH₂-CH₂-,
Sp indépendamment en chaque occurrence, représente un groupe aliphatique linéaire ou ramifié ou aromatique, en C₂-C₂₀, en particulier en C₄-C₁₈, de préférence en C₈-C₁₆ et mieux encore en C₁₀-C₁₄, qui peut être interrompu par un ou des chaînons symbolisé(s) par -O-, -CO-O-, -O-CO-, -CO-NR³-, -NR³-CO-, -O-CO-NR³-ou -NR³-CO-O, ou encore un groupe oligomère présentant une masse molaire de 400 à 1000 g/mol,
X indépendamment en chaque occurrence, ne représente rien ou représente un ou des chaînons symbolisé(s) par -O-, -CO-O-, -O-CO-, -CO-NR³-, -NR³-CO-, -O-CO-NR³- ou -NR³-CO-O,
Z indépendamment en chaque occurrence, représente un groupe polymérisable choisi parmi les groupes de formule CH₂=CR¹-CO-O- ou CH₂=CR¹-CO-NR²-,
R¹, R² et R³ représentent chacun, indépendamment en chaque occurrence, un atome d'hydrogène ou un groupe alkyle en C₁-C₇, en particulier un atome d'hydrogène ou un groupe alkyle en C₁-C₅, et de préférence un atome d'hydrogène ou un groupe alkyle en C₁-C₃,
R⁴ indépendamment en chaque occurrence, représente un groupe alkyle en C₁-C₇, en particulier un groupe alkyle en C₁-C₅, de préférence un groupe alkyle en C₁-C₃, et avec une préférence particulière, un groupe méthyle, éthyle ou isopropyle,
l'indice m peut valoir 2, 3 ou 4, mais vaut de préférence 2,
l'indice n, indépendamment en chaque occurrence, peut valoir 1 ou 2, mais vaut de préférence 1,
et l'indice p peut valoir 0, 1, 2 ou 3, mais vaut en particulier 0 ou 1, et de préférence 0..

3. Composition conforme à l'une des revendications 1 et 2, dans laquelle
R indépendamment en chaque occurrence, représente un atome d'hydrogène ou un groupe alkyle en C₁-C₅.

4. Composition conforme à l'une des revendications 1 à 3, dans laquelle
W ne représente rien ou représente un chaînon symbolisé par CH₂,
et Y, indépendamment en chaque occurrence, représente un groupe hétéroaryle ou un groupe symbolisé par -P(O)(OR⁴)₂.

5. Composition conforme à la revendication 4, dans laquelle Y, indépendamment en chaque occurrence, représente un groupe pyridyle ou un groupe symbolisé par -P(O)(OEt)₂.

6. Composition conforme à l'une des revendications précédentes, qui contient un ou plusieurs monomère(s) supplémentaire(s), polymérisable(s) par voie radicalaire, choisi(s) parmi les suivants :
(méth)acrylate de méthyle, d'éthyle, d'hydroxy-éthyle, de butyle, de benzyle, de tétrahydrofuryle ou d'isobornyle, di(méth)acrylate de bis-phénol A, bis-GMA, UDMA, di(méth)acrylate de diéthylène-glycol, de triéthylène-glycol ou de tétraéthylène-glycol, tri(méth)acrylate de tri-méthylol-propane, tétra(méth)acrylate de pentaérythritol, di(méth)acrylate de glycérol, di(méth)acrylate de butane-1,4-diol, di(méth)acrylate de décane-1,10-diol, di(méth)acrylate de dodécane-1,12-diol,
et/ou un ou plusieurs dérivés d'acrylamide dont l'atome d'azote porte un ou deux substituant(s), tels les N-éthyl-acrylamide, N,N-diméthyl-acrylamide, N-(2-hydroxy-éthyl)-acrylamide et N-méthyl-N-(2-hydroxy-éthyl)-acrylamide, ou un ou plusieurs dérivés de méthacrylamide dont l'atome d'azote porte un substituant, tels les N-éthyl-méthacrylamide et N-(2-hydroxy-éthyl)-acrylamide, de la N-vinyl-pyrrolidone, ou un ou plusieurs éther(s) d'allyle apte(s) à la réticulation,
et/ou un ou plusieurs dérivés de pyrrolidone apte(s) à la réticulation, tel le 1,6-bis(3-vinyl-pyrrolidon-2-yl)-hexane, un ou plusieurs bis-acrylamide(s) apte(s) à la réticulation, tels le méthylène-bis-acrylamide et l'éthylène-bis-acrylamide, un ou plusieurs bis(méth)acrylamide(s) apte(s) à la réticulation, tels les N,N'-diéthyl-1,3-bis(acrylamido)-propane, 1,3-bis(méthacrylamido)-propane, 1,4-bis(méthacrylamido)-butane et 1,4-bis(acryloyl)-pipérazine,
ou un mélange de ces composés.

7. Composition conforme à l'une des revendications précédentes, qui contient un ou plusieurs monomère(s) polymérisable(s) par voie radicalaire et porteur(s) de groupe acide, choisi(s) parmi les suivants :
acide maléique, acide acrylique, acide méthacrylique, acide 2-(hydroxy-méthyl)-acrylique, anhydride d'acide 4-((méth)acryloyloxy-éthyl)-tri-mellique, acide (10-méthacryloyloxy-décyl)-malonique, N-(2-hydroxy-3-méthacryloyloxy-propyl)-N-phényl-glycine, acide 4-vinyl-benzoïque, et/ou acide vinyl-phosphonique, acide (4-vinyl-phényl)-phosphonique, acide (4-vinyl-benzyl)-phosphonique, acide (2-méthacryloyloxy-éthyl)-phosphonique, acide (2-méthacrylamido-éthyl)-phosphonique, acide (4-méthacrylamido-4-méthyl-pentyl)-phosphonique, acide 2-[4-(dihydroxy-phosphoryl)-2-oxa-butyl]-acrylique, 2-[4-(dihydroxy-phosphoryl)-2-oxa-butyl]-acrylate d'éthyle ou de 2,4,6-triméthyl-phényle,
et/ou monohydrogénophosphate ou dihydrogénophosphate de 2-méthacryloyloxy-propyle, hydrogénophosphate de 2-méthacryloyloxy-éthyle et de phényle, pentaméthacryloyloxy-phosphate de dipentaérythritol, dihydrogénophosphate de 10-méthacryloyloxy-décyle, phosphate de mono(1-acryloyl-pipéridin-4-yle), dihydrogénophosphate de 6-méthacrylamido-hexyle, dihydrogénophosphate de 1,3-bis(N-acryloyl-N-propyl-amino)-prop-2-yle,
et/ou acide vinyl-sulfonique, acide (4-vinyl-phényl)-sulfonique, acide (3-méthacrylamido-propyl)-sulfonique,
ou un mélange de ces composés.

8. Composition conforme à l'une des revendications précédentes, qui contient un amorceur de polymérisation par voie radicalaire.

9. Composition conforme à l'une des revendications précédentes, qui contient un adjuvant libérant un gaz par voie thermique.

10. Composition conforme à l'une des revendications précédentes, qui contient un adjuvant capable de convertir en chaleur un rayonnement électromagnétique incident.

11. Composition conforme à l'une des revendications précédentes, qui contient une charge organique et/ou inorganique.

12. Composition conforme à l'une des revendications précédentes, qui contient
a) de 0,1 à 50 % en poids de composé de formule I,
b) de 0,01 à 10 % en poids d'amorceur,
c) de 0 à 80 % en poids de co-monomère,
d) de 0 à 80 % en poids de charge,
e) de 0 à 70 % en poids de solvant,
f) et de 0 à 10 % en poids d'adjuvant.

13. Composition conforme à la revendication 11 ou 12, qui contient de 0 à 20 % en poids de charge.

14. Composition conforme à la revendication 11 ou 12, qui contient de 20 à 80 % en poids de charge.

15. Composition conforme à l'une des revendications 1 à 14 pour utilisation en tant que matériau de dentisterie.

16. Utilisation d'une composition conforme à l'une des revendications 1 à 14 comme adhésif, composite ou matériau de stéréolithographie, ou pour la fabrication d'une pièce moulée, d'un objet en duroplaste, ou d'un élément de montage ou de consolidation.

17. Utilisation d'un composé de formule I, tel que défini dans l'une des revendications 1 à 5, pour la préparation d'un matériau de dentisterie.

18. Utilisation d'un composé de formule I, tel que défini dans l'une des revendications 1 à 5, pour la préparation d'un adhésif, d'un composite ou d'un matériau de stéréolithographie, ou pour la fabrication d'une pièce moulée, d'un objet en duroplaste, ou d'un élément de montage ou de consolidation.
